# EUROPEAN PATENT APPLICATION

(11) **EP 4 834 375 A1**
(43) Date of publication of application: **04.11.2026**
(21) Application number: 25172835.8
(22) Date of filing: 28.04.2025
(51) Int. Cl.: A61K 51/04, C07K 5/103, C07K 7/02

(54) **(4-([1,1'-BIPHENYL]-3-YLMETHOXY)-2-(PYRIDIN-2-YLOXY)PHENYL)METHANAMINE DERIVATIVES**

(71) Applicant: Helmholtz-Zentrum Dresden - Rossendorf e.V., 01328 Dresden (DE)
(72) Inventor: STADLBAUER, Sven, 01328 Dresden (DE); SACHSE, Frederik, 01328 Dresden (DE); DONAT, Cornelius, 01328 Dresden (DE); KOPKA, Klaus, 01328 Dresden (DE)
(74) Representative: Riechelmann & Carlsohn Patentanwälte PartG mbB

(57) **Abstract**

The invention relates to a compound of general formula I 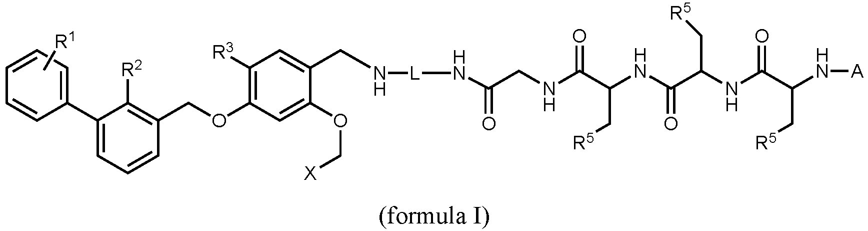 wherein
R¹ is selected from the group consisting of H, halogen, C₁-C₄ alkyl and C₁-C₄ alkoxy;
R² is selected from the group consisting of H, halogen, C₁-C₄ alkyl and C₁-C₄ alkoxy;
R³ is selected from the group consisting of H, halogen, C₁-C₄ alkyl and C₁-C₄ alkoxy;
X is a group of formula X1 or a group of formula X2

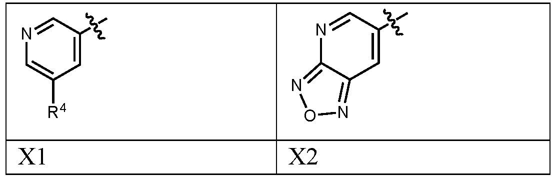

R⁴ is selected from the group consisting of -S(O)₂-C₁-C₄ alkyl, halogen and CN;
R⁵ are independently of each other selected from the group consisting of -S(O)₂OH, -P(O)(OH)₂ and -NH₂;
L is a linker selected from the group consisting of

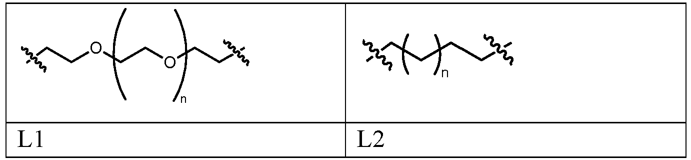

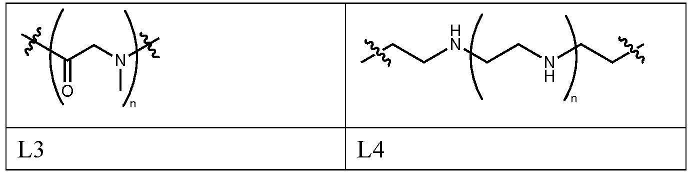

A is a chelator selected from the group consisting of

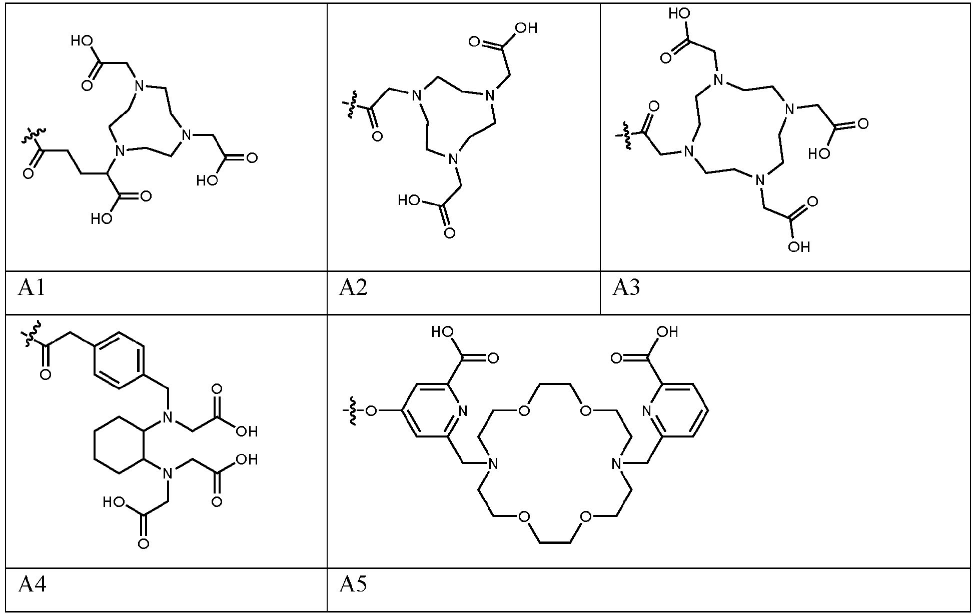

n is an integer from 1 to 5.

## Description

The invention relates to (4-([1,1'-biphenyl]-3-ylmethoxy)-2-(pyridin-2-yl-oxy)phenyl)methanamine derivatives. Further, it relates to complexes consisting of the (4-([1,1'-biphenyl]-3-ylmethoxy)-2-(pyridin-2-yloxy)phenyl)methanamine derivatives and a radionuclide and uses of the (4-([1,1'-biphenyl]-3-ylmethoxy)-2-(pyridin-2-yl-oxy)phenyl)methanamine derivatives in the preparation of a radiopharmaceutical agent.

Programmed Death Receptor 1 (CD279, PD-1) is a receptor on T cells that is inactivated by binding to its ligand (PD-L1, CD274, B7-H1). PD-1 / PD-ligand interactions regulate immune responses, usually during the resolution of an infection or in the context of developing self-tolerance. Stimulation, for example in tumor diseases or chronic infections, leads to increased expression of PD-1 in the T cells. These T-cells attack the virus or tumor cells. Tumor cells over-expressing the corresponding ligand thus have a selection advantage and can escape the local immune response. Exposure to the corresponding antigen over a longer period of time can cause so-called "T-cell exhaustion". This then leads to a reduced immune response in and around the tumor and thus promotes further growth and proliferation. This blockade of the PD-1 / PD-L1 interaction can be broken by the use of antibodies, e.g. against PD-1 or PD-L1, and results in a reactivation of the T cells. Ideally, this reactivation of the immune system allows the local immune response to attack tumor cells and prevent growth and proliferation. Cancer patients can benefit from antibody therapy directed against PD-1 or PD-L1 just as much as patients with a viral infection. However, the response rate with antibody monotherapy is only 30%. In addition to the antibodies currently in clinical use, peptides and small molecule organic compounds that bind to and inhibit PD-L1 have also been developed. These compounds are in preclinical testing. ^{[1-5]} Two small molecule inhibitors, GS-4224 ^{[6]} and INCB086550 ^{[7]}, are currently in Phase I and II clinical trials. A major difference between these small molecule inhibitors and antibodies/ peptides is the postulated binding mode. Upon binding of the small molecules, dimerization of two PD-L1 proteins occurs, followed by internalization of the inhibitor-PD-L1 complex. ^{[8,9]} For therapy, this internalization is one of the main advantages over previously used antibodies.

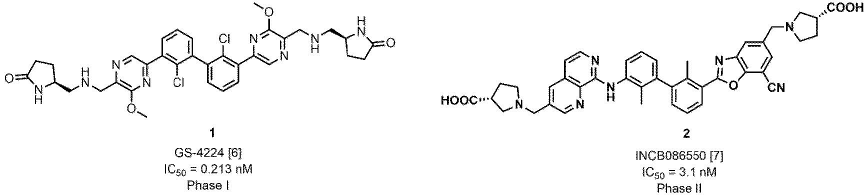

The above compounds **GS-4224** and **INCB086550** are examples for patented non-peptidic compounds of low molecular weight for immune checkpoint inhibitor therapy examined in clinical studies.

The low-molecular weight organic molecules described are all structurally similar (see scheme 1).^{[10]} They have a biaryl moiety (section 1 in scheme S1), which is particularly important for binding affinity towards the target. In addition, the central chloroaromatic (section 2 in scheme S1) and an ether-bridged cyanopyridyl group (section 3 in scheme S1) also play a role in binding. Water solubility is ensured by amino alcohols or amino acids (section 4 in scheme S1). An optional extension unit (section 5 in scheme S1) can further add to the binding affinity. Despite the presence of amino acids, the substances reported to date do not exhibit water solubility, but rather a very high lipophilicity, which makes them difficult to use in imaging and endoradiotherapy.

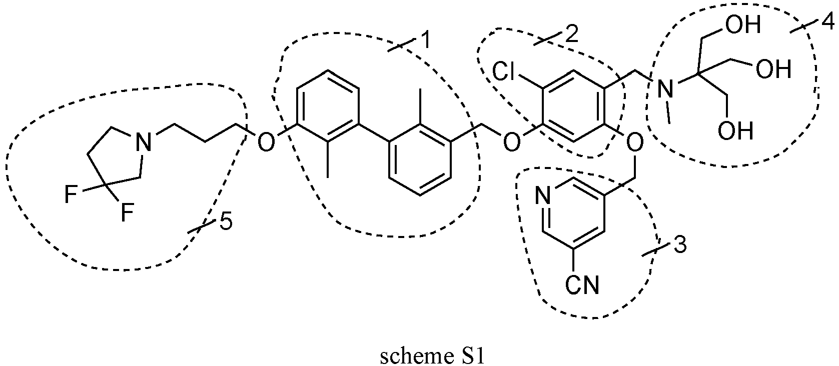

A decision on the use of immune checkpoint inhibitor therapy is made by determining PD-L1 protein expression using immunohistochemical methods on biopsy resectates. PD-L1 expression can be heterogeneous, both between and within tumor lesions, making it difficult for the treating oncologist to decide on a therapy. Non-invasive molecular imaging techniques such as Positron Emission Tomography (PET) and Single Photon Emission Computed Tomography (SPECT), are better suited to select patients who are likely to respond prior to therapy (patient stratification), as they can address the problem of heterogeneous expression of PD-L1 over time. For this purpose, PD-L1-affine low molecular weight organic molecules, peptides and antibodies can be labeled with a radionuclide (e.g. fluorine-18, gallium-68, iodine-123), which allows imaging of PD-L1 expression. To date, PD-L1-affine antibodies, nanobodies and affibodies have been radiolabeled and studied both preclinically and clinically as PET or SPECT tracers.^{[11-15]}

Some peptidic ligands have been successfully tested preclinically in tumor-bearing animals,^{[16-20]} while another has been tested in a clinical trial in nine patients.^{[21]} Low molecular weight organic molecules have also been developed and tested preclinically after labeling with various PET and SPECT nuclides in small animals.^{[22-28]} A Chinese patent, published in 2020 describes the radiolabeling of various low molecular weight PD-L1 binders with different radioactive iodine isotopes ^{[29]}: 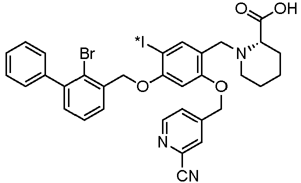 wherein *I is 1-123, 1-124, 1-125, I-129, or I-131.

A disadvantage of radiolabeled antibodies, affibodies and nanobodies is their potential immunogenicity, which can cause adverse immunological effects such as cytokine storms.^{[30]} A disadvantage of antibody-based radiotracers for imaging is the long circulation time of several days before the maximum accumulation in the tumor is reached for good imaging contrast, making radiolabeled antibodies less desirable as imaging agents. In contrast, peptides and small organic molecules have the advantage of higher tissue penetration and faster accumulating in the tumor tissue, allowing maximum tumor uptake in the range of minutes to hours. In addition, these short circulation times allow labeling with PET nuclides such as carbon-11 and fluorine-18, which offer a particularly good resolution of the PET images. However, the small molecules described so far show a comparatively lower tumor uptake and, due to their high lipophilicity and low water solubility, an increased nonspecific binding and excretion through the liver.L^{[22-28]}

The problem of the invention is to eliminate the drawbacks of the prior art. In particular, (4-([1,1'-biphenyl]-3-ylmethoxy)-2-(pyridin-2-yloxy)phenyl)methanamine derivatives shall be provided that bind to PD-L1, but have a lower lipophilicity and a higher water solubility. In particular, (4-([1,1'-biphenyl]-3-ylmethoxy)-2-(pyridin-2-yloxy)phenyl)methanamine derivatives shall be provided that after radioactive labelling with a radionuclide are suitable for the molecular non- and minimal-invasive nuclear-medical imaging and endoradiotherapy of tumor diseases into which the Programmed Death Receptor Ligand 1 (PD-L1) is involved. Moreover, (4-([1,1'-biphenyl]-3-ylmethoxy)-2-(pyridin-2-yloxy)phenyl)methanamine derivatives shall be provided that are suitable for the diagnosis and therapy of non-small cell lung carcinoma.

This problem is solved by the features of claims 1, 8, 11, 12, 13, and 15. Suitable developments of the inventions result from the features of the dependent claims.

According to the invention a compound of general formula I is provided: 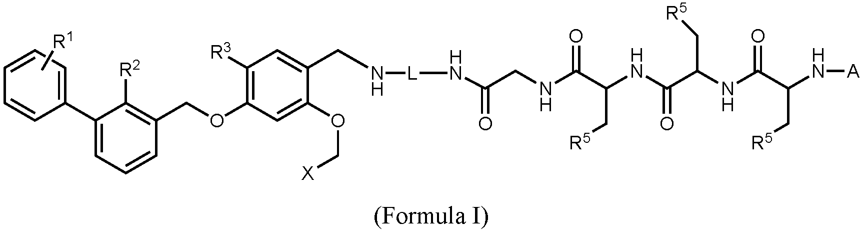 wherein
R¹ is selected from the group consisting of H, halogen, C₁-C₄ alkyl and C₁-C₄ alkoxy;
R² is selected from the group consisting of H, halogen, C₁-C₄ alkyl and C₁-C₄ alkoxy;
R³ is selected from the group consisting of H, halogen, C₁-C₄ alkyl and C₁-C₄ alkoxy;
X is a group of formula X1 or a group of formula X2

| | |
|---|---|
| 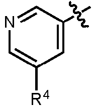 | 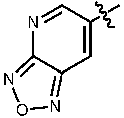 |
| X1 | X2 |

R⁴ is selected from the group consisting of -S(O)₂-C₁-C₄ alkyl, halogen and CN;
R⁵ are independently of each other selected from the group consisting of -S(O)₂OH, -P(O)(OH)₂ and -NH₂;
L is a linker selected from the group consisting of

| | |
|---|---|
| 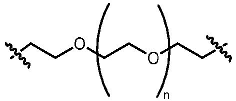 | 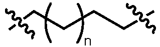 |
| L1 | L2 |
| 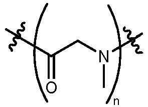 | 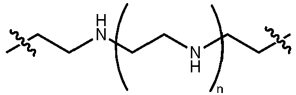 |
| L3 | L4 |

A is a chelator selected from the group consisting of

| | | |
|---|---|---|
| 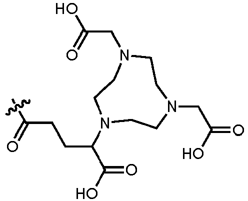 | 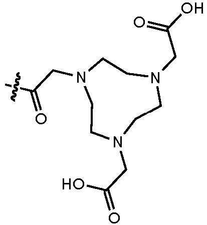 | 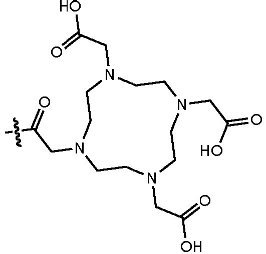 |
| A1 | A2 | A3 |
| 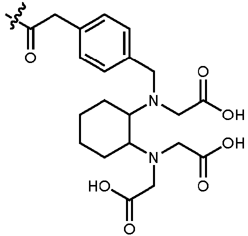 | 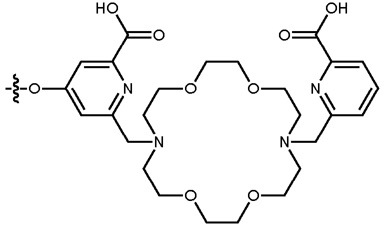 | |
| A4 | A5 | |

n is an integer from 1 to 5.

The compounds according to the invention are PD-L1-addressing compounds. The compounds according to the invention bind to PD-L1. The compounds according to the invention are particularly suitable for the diagnostics and/or therapy of diseases, into which the Programmed Death Receptor Ligand 1 (PD-L1) is involved.

According to the invention R¹ is selected from the group consisting of H, halogen, C₁-C₄ alkyl and C₁-C₄ alkoxy. Preferably, R¹ is selected from the group consisting of H, halogen or C₁-C₄ alkyl. More preferred R¹ is selected from the group consisting of H, halogen or methyl. Particularly preferred R¹ is selected from the group consisting of H, bromine, chlorine or methyl. Most preferred R¹ is H or methyl.

According to the invention R² is selected from the group consisting of H, halogen, C₁-C₄ alkyl and C₁-C₄ alkoxy. Preferably, R² is selected from the group consisting of H, halogen or C₁-C₄ alkyl. More preferred R² is selected from the group consisting of chlorine, bromine or methyl. Most preferred R² is bromine or methyl.

According to the invention R³ is selected from the group consisting of H, halogen, C₁-C₄ alkyl and C₁-C₄ alkoxy. Preferably, R³ is selected from the group consisting of H, halogen or C₁-C₄ alkyl. More preferred R³ is selected from the group consisting of chlorine, bromine or methyl. Most preferred R³ is chlorine or methyl.

According to the invention X is a group of formula X1 or a group of formula X2. Preferably, X is a group of formula X1. More preferred X is a group of formula X1 and R⁴ is selected from the group consisting -S(O)₂-C₁-C₄ alkyl, halogen and CN. Particularly preferred X is a group of formula X1 and R⁴ is -S(O)₂-C₁-C₄ alkyl. Most preferred X is a group of formula X1 and R⁴ is -S(O)₂-methyl.

According to the invention R⁵ are independently of each other selected from the group consisting of -S(O)₂OH, -P(O)(OH)₂ and -NH₂. Preferably, R⁵ are independently of each other -S(O)₂OH or -P(O)(OH)₂. More preferred all R⁵ are -S(O)₂OH.

According to the invention L is a linker selected from the group of consisting of L1, L2, L3 and L4. Preferably, L is a linker selected from the group of consisting of L1, L2 and L3 and n is an integer from 1 to 5, preferred 1 to 3. More preferred L is L1 or L2. Particularly preferred L is L1 or L2 and n is an integer from 1 to 5, preferred 1 to 3. Most preferred L is L1 and n is an integer from 1 to 3, preferred 1.

According to the invention A is a chelator selected from the group of consisting of A1, A2, A3, A4 and A5. Preferably, A is a chelator selected from the group of consisting of A1, A2, A3 and A5. More preferred A is a chelator selected from the group of consisting of A1, A3 and A5. Most preferred A is A1.

There may be provided a compound of general formula I, wherein
R¹ is selected from the group consisting of H, halogen or C₁-C₄ alkyl;
R² is selected from the group consisting of H, halogen or C₁-C₄ alkyl;
R³ is selected from the group consisting of H, halogen or C₁-C₄ alkyl;
X is a group of formula X1;
R⁴ is -S(O)₂-C₁-C₄ alkyl;
R⁵ are independently of each other -S(O)₂OH or -P(O)(OH)₂;
L is a linker selected from the group of consisting of L1, L2 and L3 and n is an integer from 1 to 5, preferred 1 to 3; and
A is a chelator selected from the group consisting of A1, A2, A3 and A5.

There may be provided a compound of general formula I, wherein
R¹ is selected from the group consisting of H, halogen or methyl;
R² is selected from the group consisting of chlorine, bromine or methyl;
R³ is selected from the group consisting of chlorine, bromine or methyl;
X is a group of formula X1;
R⁴ is -S(O)₂-C₁-C₄ alkyl or halogen;
all R⁵ are -S(O)₂OH;
L is L1 or L2 and n is an integer from 1 to 5, preferred 1 to 3; and
A is a chelator selected from the group of consisting of A1, A3 and A5.

There may be provided a compound of general formula I, wherein
R¹ is selected from the group consisting of H or methyl;
R² is selected from the group consisting of bromine or methyl;
R³ is selected from the group consisting of chlorine or methyl;
X is a group of formula X1;
R⁴ is -S(O)₂-C₁-C₄ alkyl;
all R⁵ are -S(O)₂OH;
L is L1 or L2 and n is an integer from 1 to 3, preferred 1; and
A is A1 and A5.

There may be provided a compound of general formula I, wherein
R¹ is selected from the group consisting of H or methyl;
R² is selected from the group consisting of bromine or methyl;
R³ is selected from the group consisting of chlorine or methyl;
X is a group of formula X1;
R⁴ is -S(O)₂-methyl;
all R⁵ are -S(O)₂OH;
L is L1 and n is an integer from 1 to 3, preferred 1; and
A is A1.

There may be provided a compound of general formula I, wherein
R¹ is selected from the group consisting of H;
R² is selected from the group consisting of bromine;
R³ is selected from the group consisting of chlorine;
X is a group of formula X1;
R⁴ is -S(O)₂-methyl;
all R⁵ are -S(O)₂OH;
L is L1 and n is 1; and
A is A1.

The most preferred compounds according to the invention are

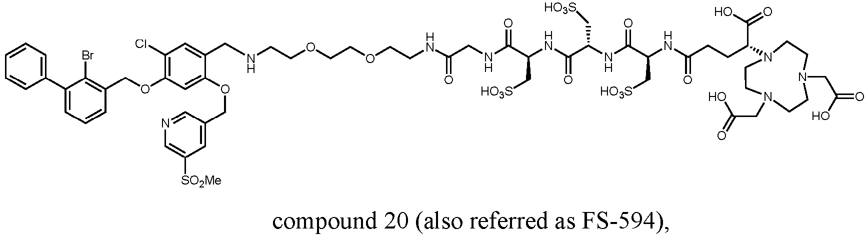

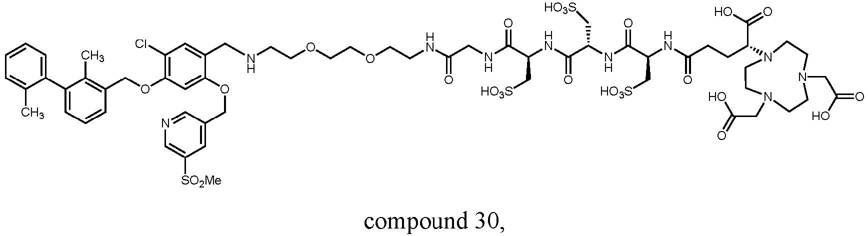

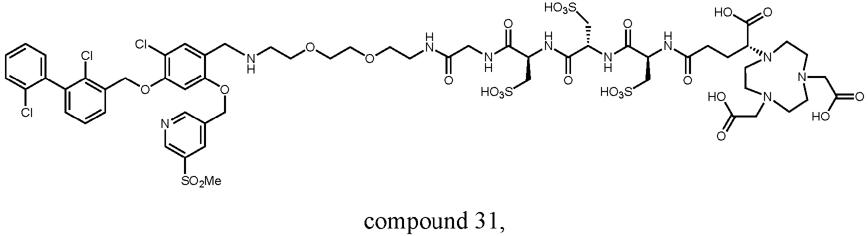

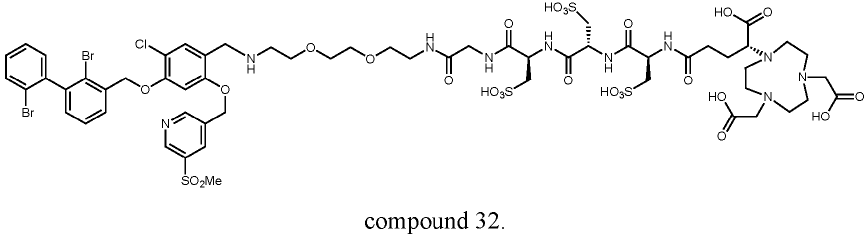

In another embodiment according to the invention X is preferably a group of formula X2.

According to this embodiment of the invention R¹ is selected from the group consisting of H, halogen, C₁-C₄ alkyl and C₁-C₄ alkoxy. Preferably, R¹ is selected from the group consisting of H, halogen or C₁-C₄ alkyl. More preferred R¹ is selected from the group consisting of H, halogen or methyl. Particularly preferred R¹ is selected from the group consisting of H, bromine, chlorine or methyl. Most preferred R¹ is H or methyl.

According to this embodiment of the invention R² is selected from the group consisting of H, halogen, C₁-C₄ alkyl and C₁-C₄ alkoxy. Preferably, R² is selected from the group consisting of H, halogen or C₁-C₄ alkyl. More preferred R² is selected from the group consisting of chlorine, bromine or methyl. Most preferred R² is bromine or methyl.

According to this embodiment of the invention R³ is selected from the group consisting of H, halogen, C₁-C₄ alkyl and C₁-C₄ alkoxy. Preferably, R³ is selected from the group consisting of H, halogen or C₁-C₄ alkyl. More preferred R³ is selected from the group consisting of chlorine, bromine or methyl. Most preferred R³ is chlorine or methyl.

According to this embodiment of the invention R⁵ are independently of each other selected from the group consisting of -S(O)₂OH, -P(O)(OH)₂ and -NH₂. Preferably, R⁵ are independently of each other -S(O)₂OH or -P(O)(OH)₂. More preferred all R⁵ are -S(O)₂OH.

According to this embodiment of the invention L is a linker selected from the group of consisting of L1, L2, L3 and L4. Preferably, L is a linker selected from the group of consisting of L1, L2 and L3 and n is an integer from 1 to 5, preferred 1 to 3. More preferred L is L1 or L2. Particularly preferred L is L1 or L2 and n is an integer from 1 to 5, preferred 1 to 3. Most preferred L is L1 and n is an integer from 1 to 3, preferred 1.

According to this embodiment of the invention A is a chelator selected from the group consisting of A1, A2, A3, A4 and A5. Preferably, A is a chelator selected from the group of consisting of A1, A2, A3 and A5. More preferred A is a chelator selected from the group of consisting of A1, A3 and A5. Most preferred A is A1.

There may be provided a compound of general formula I, wherein
R¹ is selected from the group consisting of H, halogen or C₁-C₄ alkyl;
R² is selected from the group consisting of H, halogen or C₁-C₄ alkyl;
R³ is selected from the group consisting of H, halogen or C₁-C₄ alkyl;
X is a group of formula X2;
R⁵ are independently of each other -S(O)₂OH or -P(O)(OH)₂;
L is a linker selected from the group consisting of L1, L2 and L3 and n is an integer from 1 to 5, preferred 1 to 3; and
A is a chelator selected from the group of consisting of A1, A2, A3 and A5.

There may be provided a compound of general formula I, wherein
R¹ is selected from the group consisting of H, halogen or methyl;
R² is selected from the group consisting of chlorine, bromine or methyl;
R³ is selected from the group consisting of chlorine, bromine or methyl;
X is a group of formula X2;
all R⁵ are -S(O)₂OH;
L is L1 or L2 and n is an integer from 1 to 5, preferred 1 to 3; and
A is a chelator selected from the group of consisting of A1, A3 and A5.

There may be provided a compound of general formula I, wherein
R¹ is selected from the group consisting of H or methyl;
R² is selected from the group consisting of bromine or methyl;
R³ is selected from the group consisting of chlorine or methyl;
X is a group of formula X2;
all R⁵ are -S(O)₂OH;
L is L1 or L2 and n is an integer from 1 to 3, preferred 1; and
A is A1 and A5.

There may be provided a compound of general formula I, wherein
R¹ is selected from the group consisting of H or methyl;
R² is selected from the group consisting of bromine or methyl;
R³ is selected from the group consisting of chlorine or methyl;
X is a group of formula X2;
all R⁵ are -S(O)₂OH;
L is L1 and n is an integer from 1 to 3, preferred 1; and
A is A1.

There may be provided a compound of general formula I, wherein
R¹ is selected from the group consisting of H;
R² is selected from the group consisting of bromine;
R³ is selected from the group consisting of chlorine;
X is a group of formula X2;
all R⁵ are -S(O)₂OH;
L is L1 and n is 1; and
A is A1.

The most preferred compounds according to this embodiment of the invention are 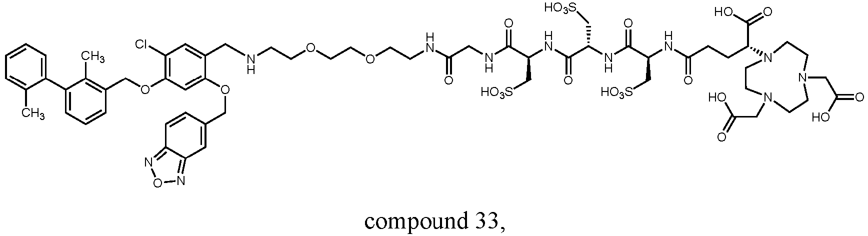 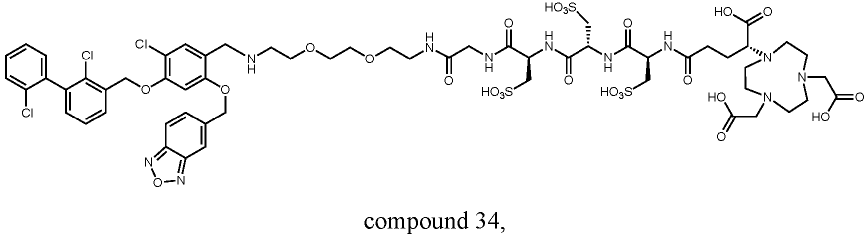 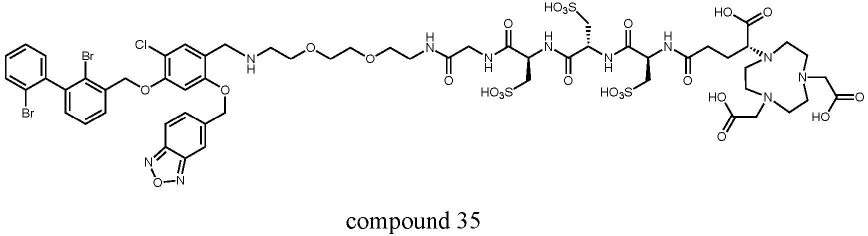 and

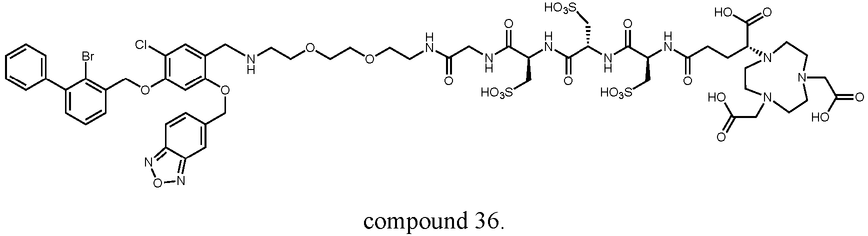

In one embodiment the compounds according to the invention are radiotracers. The radiotracers can be used for the diagnostics and/or therapy of cancer diseases in which the Programmed Death Receptor Ligand 1 (PD-L1) is involved. The radiotracers are PD-L1-addressing radiotracers. The radiotracers are complexes consisting of a compound according to the invention and a radionuclide.

According to the invention there is be provided a complex consisting of a compound of general formula I and a radioactive metal ion or Al[¹⁸F]F²⁺. Here, the radioactive metal ion is preferably selected from the group consisting of [⁶¹Cu]Cu²⁺, [⁶⁴Cu]Cu²⁺, [⁶⁷Cu]Cu²⁺, [⁶⁷Ga]Ga³⁺, [⁶⁸Ga]Ga³⁺, [¹⁷⁷Lu]Lu³⁺, [¹⁴⁹Tb]Tb³⁺, [¹⁵⁵Tb]Tb³⁺, [¹⁵²Tb]Tb³⁺, [¹⁶¹Tb]Tb³⁺, [²²⁵Ac]Ac³⁺, [²²⁶Ac]Ac³⁺, [¹³²La]La³⁺, [¹³³La]La³⁺, [¹³⁵La]La³⁺, [¹¹¹In]In³⁺, [⁸⁶Y]Y³⁺, [⁹⁰Y]Y³⁺ [²¹²Bi]Bi³⁺, [²¹³Bi]Bi³⁺, [⁴³Sc]Sc³⁺, [⁴⁴Sc]Sc³⁺, [⁴⁷Sc]Sc³⁺, [²⁰³Pb]Pb³⁺ and [²¹²Pb]Pb³⁺.

The substances according to the invention permit the immediate (minutes to hours after injection) imaging of PD-L1 overexpressing tumors. This means that the patient can be examined on the same day using PET/CT, SPECT/CT or PET/MR. This eliminates the disadvantage of antibody-based radiotracers, which have circulation times of several days. In addition, the substances exhibit lower liver uptake and promote excretion via the kidneys. All this reduces the patient's exposure to radiation and enables optimized clinical examinations. The almost complete internalization of the substances also allows therapeutic application in the form of endoradiotherapy with alpha and beta emitters or Meitner-Auger electron emitters. This is a decisive advantage over antibodies and peptides which bind to a large extent to the cell surface and are typically not fully internalized.

There may be provided a complex consisting of compound of general formula I, wherein A is A1, and Al[¹⁸F]F²⁺ or the radioactive metal ion selected from the group consisting of [⁶¹Cu]Cu²⁺, [⁶⁴Cu]Cu²⁺, [⁶⁷Cu]Cu²⁺, [⁶⁷Ga]Ga³⁺, [⁶⁸Ga]Ga³⁺, [⁴³Sc]Sc³⁺, [⁴⁴Sc]Sc³⁺, [⁴⁷Sc]Sc³⁺. Preferred is a complex consisting of compound of general formula I, wherein A is A1, and the radioactive metal ion selected from the group consisting of [⁶⁴Cu]Cu²⁺, [⁶⁸Ga]Ga³⁺ and [⁴⁴Sc]Sc³⁺. More preferred is a complex consisting of compound of general formula I, wherein A is A1, and the radioactive metal ion selected from the group consisting of [⁶⁴Cu]Cu²⁺ and [⁶⁸Ga]Ga³⁺. Most preferred is a complex consisting of compound of general formula I, wherein A is A1, and the radioactive metal ion [⁶⁴Cu]Cu²⁺.

There may be provided a complex consisting of compound of general formula I, wherein A is A2, and Al[¹⁸F]F²⁺ or the radioactive metal ion selected from the group consisting of [⁶¹Cu]Cu²⁺, [⁶⁴Cu]Cu²⁺, [⁶⁷Cu]Cu²⁺, [⁶⁷Ga]Ga³⁺, [⁶⁸Ga]Ga³⁺, [⁴³Sc]Sc³⁺, [⁴⁴Sc]Sc³⁺, [⁴⁷Sc]Sc³⁺. Preferred is a complex consisting of compound of general formula I, wherein A is A2, and the radioactive metal ion selected from the group consisting of [⁶⁴Cu]Cu²⁺, [⁶⁸Ga]Ga³⁺ and [⁴⁴Sc]Sc³⁺. More preferred is a complex consisting of compound of general formula I, wherein A is A2, and the radioactive metal ion selected from the group consisting of [⁶⁴Cu]Cu²⁺ and [⁶⁸Ga]Ga³⁺. Most preferred is a complex consisting of compound of general formula I, wherein A is A2, and the radioactive metal ion [⁶⁴Cu]Cu²⁺.

There may be provided a complex consisting of compound of general formula I, wherein A is A3, and the radioactive metal ion selected from the group consisting of [⁶¹Cu]Cu²⁺, [⁶⁴Cu]Cu²⁺, [⁶⁷Cu]Cu²⁺, [⁶⁷Ga]Ga³⁺, [⁶⁸Ga]Ga³⁺, [¹⁷⁷Lu]Lu³⁺, [¹⁴⁹Tb]Tb³⁺, [¹⁵⁵Tb]Tb³⁺, [¹⁵²Tb]Tb³⁺, [¹⁶¹Tb]Tb³⁺, [²²⁵Ac]Ac³⁺, [²²⁵Ac]Ac³⁺, [¹³²La]La³⁺, [¹³³La]La³⁺, [¹³⁵La]La³⁺ [¹¹¹In]In³⁺, [⁸⁶Y]Y³⁺, [⁹⁰Y]Y³⁺, [²¹²Bi]Bi³⁺, [²¹³Bi]Bi³⁺, [⁴³Sc]Sc³⁺, [⁴⁴Sc]Sc³⁺, [⁴⁷Sc]Sc³⁺, [²⁰³Pb]Pb³⁺ and [²¹²Pb]Pb³⁺. Preferred is a complex consisting of compound of general formula I, wherein A is A3, and the radioactive metal ion selected from the group consisting of [⁶⁴Cu]Cu²⁺, [⁶⁸Ga]Ga³⁺ and [⁴⁴Sc]Sc³⁺. More preferred is a complex consisting of compound of general formula I, wherein A is A3, and the radioactive metal ion selected from the group consisting of [⁶⁴Cu]Cu²⁺ and [⁶⁸Ga]Ga³⁺. Most preferred is a complex consisting of compound of general formula I, wherein A is A3, and the radioactive metal ion [⁶⁴Cu]Cu²⁺.

There may be provided a complex consisting of compound of general formula I, wherein A is A4, and Al[¹⁸F]F²⁺.

There may be provided a complex consisting of compound of general formula I, wherein A is A5, and the radioactive metal ion selected from the group consisting of [¹⁷⁷Lu]Lu³⁺, [¹⁴⁹Tb]Tb³⁺, [¹⁵⁵Tb]Tb³⁺, [¹⁵²Tb]Tb³⁺, [¹⁶¹Tb]Tb³⁺, [²²⁵Ac]Ac³⁺, [²²⁶Ac]Ac³⁺, [¹³²La]La³⁺, [¹³³La]La³⁺, [¹³⁵La]La³⁺, [²¹²Bi]Bi³⁺, [²¹³Bi]Bi³⁺, [²⁰³Pb]Pb³⁺ and [²¹²Pb]Pb³⁺. Preferred is a complex consisting of compound of general formula I, wherein A is A5, and the radioactive metal ion selected from the group consisting of [¹⁷⁷Lu]Lu³⁺, [²²⁵Ac]Ac³⁺, [¹³³La]La³⁺ and [²¹²Pb]Pb³⁺. More preferred is a complex consisting of compound of general formula I, wherein A is A5, and the radioactive metal ion selected from the group consisting of [²²⁵Ac]Ac³⁺ and [²¹²Pb]Pb³⁺. Most preferred is a complex consisting of compound of general formula I, wherein A is A5, and the radioactive metal ion [²²⁵Ac]Ac³⁺.

In a more preferred embodiment, there is provided a complex
consisting of a compound of general formula I, wherein R¹ is selected from the group consisting of H or methyl; R² is selected from the group consisting of bromine or methyl; R³ is selected from the group consisting of chlorine or methyl; X is a group of formula X1; R⁴ is -S(O)₂-methyl; all R⁵ are -S(O)₂OH; L is L1 and n is an integer from 1 to 3, preferred 1; and A is A1, and
the radioactive metal ion [⁶⁴Cu]Cu²⁺ or [⁶⁸Ga]Ga³⁺.

In a further preferred embodiment, there is provided a complex
consisting of a compound of general formula I, wherein R¹ is H; R² is bromine or methyl; R³ is chlorine or methyl; X is a group of formula X1; R⁴ is -S(O)₂-methyl; all R⁵ are -S(O)₂OH; L is L1 and n is an integer from 1 to 3, preferred 1; and A is A1, and the radioactive metal ion [⁶⁴Cu]Cu²⁺ or [⁶⁸Ga]Ga³⁺.

In the most preferred embodiment, there is provided a complex
consisting of a compound of general formula I, wherein R¹ is H; R² is bromine; R³ is chlorine; X is a group of formula X1; R⁴ is -S(O)₂-methyl; all R⁵ are -S(O)₂OH; L is L1 and n is 1; and A is A1, and the radioactive metal ion [⁶⁴Cu]Cu²⁺.

According to the invention a complex is a structure, in which a central atom (here a radioactive metal ion or Al[¹⁸F]F²⁺) is surrounded by a molecule that is referred as ligand (here the entity A).

The compounds according to the invention each have water-soluble groups. These groups are the residue R⁵. Residues R⁵ are preferably sulphonic acid groups and phosphonic acid groups that replace the amino acid groups and amino alcohol groups known from the prior art. Thus, the compounds according to the invention in contrast to the compounds known from the prior art possess a low lipophilicity. It has been found that moiety R⁵ has no detrimental influence on the binding affinity to the PD-L1 target structure.

In summary, the present invention is based on low-molecular, non-peptidic substances which are suitable for the diagnosis and endoradiotherapy of PD-L1 positive tumors via introduced chelators with radiometals as defined above. In order to overcome the problem of the high lipophilicity of the leading structures known in the literature, the molecules were modified with water-soluble groups without, however, losing the binding affinity to the PD-L1 target structure. The invention therefore differs from the prior art in that water-soluble groups on the one hand and a chelator for a radiometal on the other were inserted into the molecular structures.

### Definitions

The term "alkyl", unless specified otherwise, particularly relates to a monovalent, saturated, aliphatic hydrocarbon group having a branched or unbranched carbon chain with 1 to 6 carbon atoms, preferably 1 to 4 carbon atoms, and particularly preferably 1 to 4 carbon atoms. Examples of alkyl groups include, but are not limited to methyl, ethyl, propyl, isopropyl, isobutyl, *sec*-butyl, *tert*-butyl, and the like. The alkyl group may optionally be substituted with one or more substituents, wherein each substituent independently is hydroxy, alkyl, alkoxy, aryl, halogen, haloalkyl, cyano, nitro, amino, mono-alkylamino or dialkylamino, unless specified otherwise.

The term "alkoxy", unless specified otherwise, particularly relates to a group of formula -OR, wherein R is an alkyl group, as defined herein. Examples of alkoxy components include, but are not limited to methoxy, ethoxy, isopropoxy, and the like. The alkoxy group may optionally be substituted with one or more substituents, wherein each substituent independently is hydroxy, alkyl, alkoxy, aryl, halogen, haloalkyl, cyano, nitro, amino, mono-alkylamino or dialkylamino, unless specified otherwise.

The term "halogen" relates to fluorine, chlorine, bromine, or iodine.

The term "sulphonic acid group", unless otherwise specified, relates to a group -S(O)₂OH.

Preferred Examples of a compound of general formula (I) are given in the following: 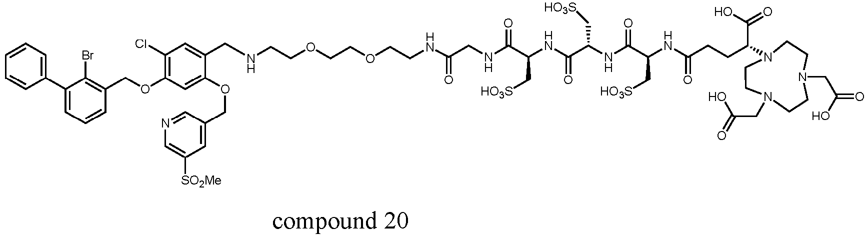 (also referred as FS-594), 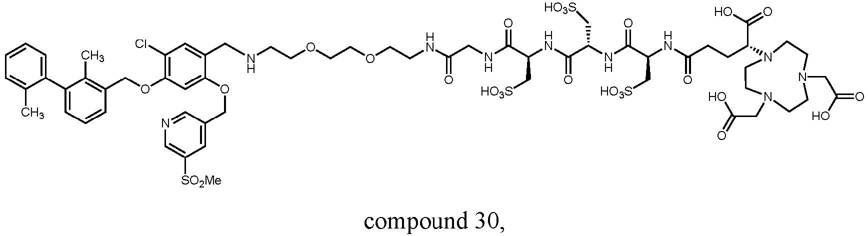 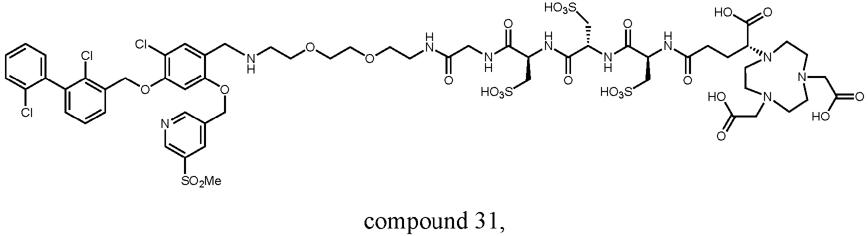 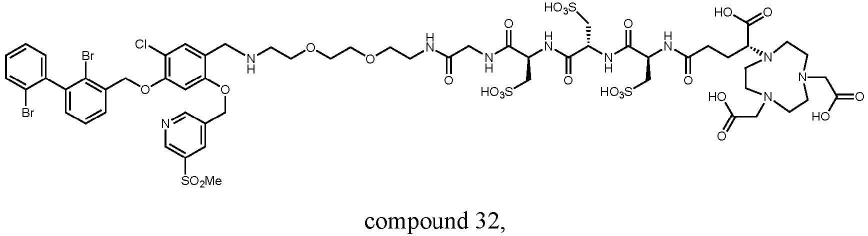 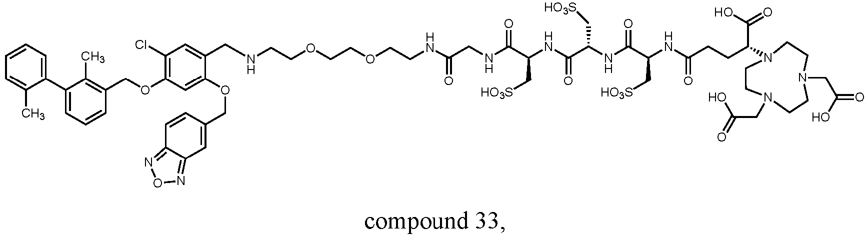 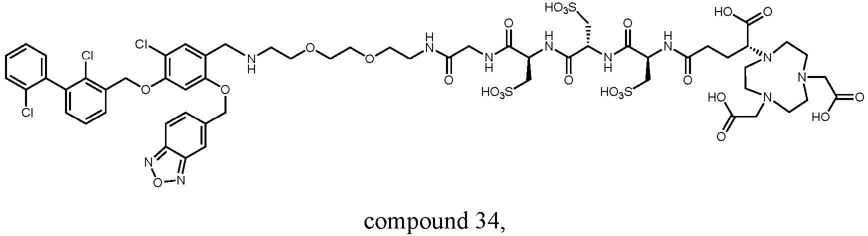 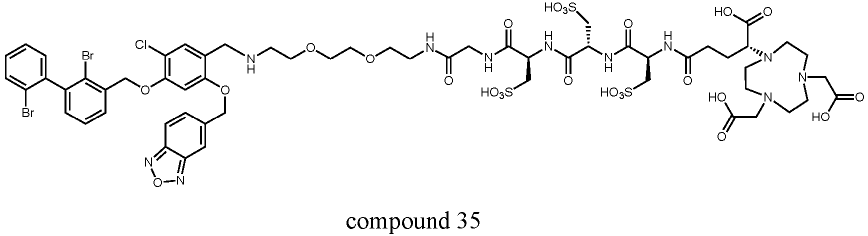 and

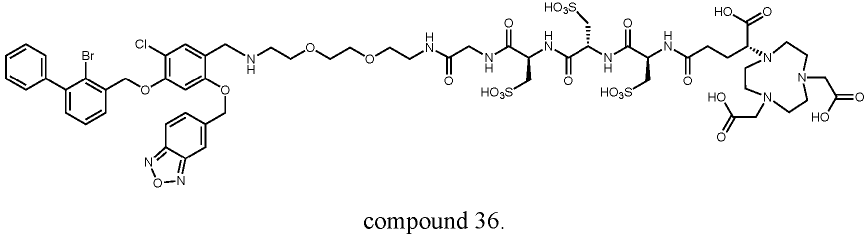

Compound **20** is a compound of general formula I, in which R¹ is hydrogen, R² is bromine, R³ is chlorine, X is a group of formula X1, wherein R⁴ is a methanesulphonic acid group, all R⁵ are sulphonic acid groups, L is a group of formula L1 with n = 1, and A is a group of formula A1.

Compound 30 is a compound of general formula I, in which R¹ is methyl, R² is methyl, R³ is chlorine, X is a group of formula X1, wherein R⁴ is a methanesulphonic acid group, all R⁵ are sulphonic acid groups, L is a group of formula L1 with n = 1, and A is a group of formula A1.

Compound **31** is a compound of general formula I, in which R¹ is chlorine, R² is chlorine, R³ is chlorine, X is a group of formula X1, wherein R⁴ is a methanesulphonic acid group, all R⁵ are sulphonic acid groups, L is a group of formula L1 with n = 1, and A is a group of formula A1.

Compound **32** is a compound of general formula I, in which R¹ is bromine, R² is bromine, R³ is chlorine, X is a group of formula X1, wherein R⁴ is a methanesulphonic acid group, all R⁵ are sulphonic acid groups, L is a group of formula L1 with n = 1, and A is a group of formula A1.

Compound **33** is a compound of general formula I, in which R¹ is methyl, R² is methyl, R³ is chlorine, X is a group of formula X2, all R⁵ are sulphonic acid groups, L is a group of formula L1 with n = 1, and A is a group of formula A1.

Compound **34** is a compound of general formula I, in which R¹ is chlorine, R² is chlorine, R³ is chlorine, X is a group of formula X2, all R⁵ are sulphonic acid groups, L is a group of formula L1 with n = 1, and A is a group of formula A1.

Compound **35** is a compound of general formula I, in which R¹ is bromine, R² is bromine, R³ is chlorine, X is a group of formula X2, all R⁵ are sulphonic acid groups, L is a group of formula L1 with n = 1, and A is a group of formula A1.

Compound **36** is a compound of general formula I, in which R¹ is H, R² is bromine, R³ is chlorine, X is a group of formula X2, all R⁵ are sulphonic acid groups, L is a group of formula L1 with n = 1, and A is a group of formula A1.

### Preparation of the compounds of general formula I

The compounds of general formula I can be prepared by reacting a base structure of formula **X** with a compound of general formula H₂N-L-NH₂ (**XIII-a**), wherein R¹, R², R³, X and L have the definitions given in claim 1 (see scheme 1) 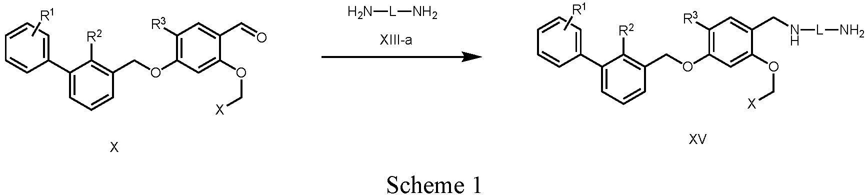 Compound **10** is an example of a compound of the base structure **X**.

In the following scheme the reaction of a compound of general formula **XV** with a compound of general formula **XVI-a** to a compound of general formula **XVIII-a** is shown. 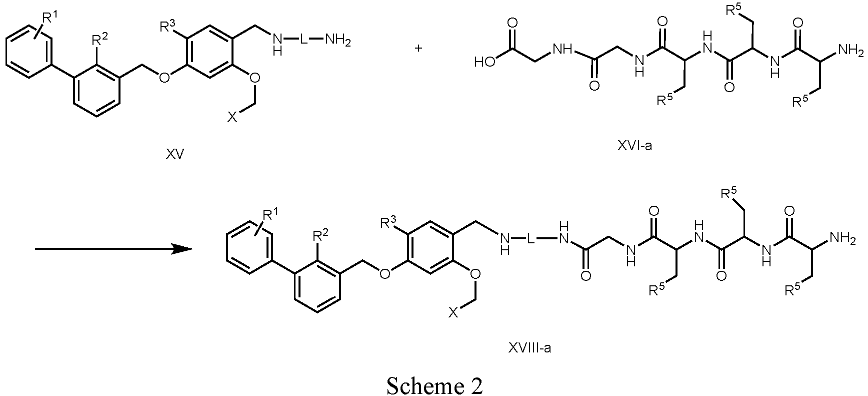 Compound **18** is an example of a compound of general formula **XVIII-a**.

In the next step the reaction of a compound of general formula **XVIII-a** with a compound of general formula **HO-A** is shown, wherein R¹, R², R³, R⁵, X, and L have the definitions given in claim 1 to a compound of general formula I, wherein R¹, R², R³, R⁵, X, L and A have the definitions given in claim 1, as is shown in scheme 3. 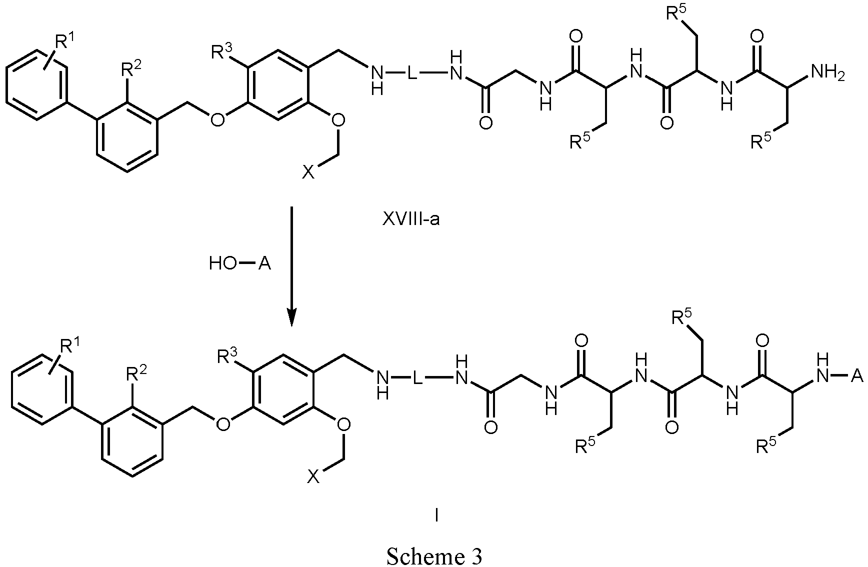 Compounds **20** and **30** to **31** are examples of a compound of general formula **I**.

Scheme 3 shows the way of preparing a compound of general formula I with a chelate ligand.

In the following, the invention is explained in detail with the help of examples not intended to limit the invention with respect to the drawings. Here,
- Fig. 1: shows a diagram as obtained in real-time binding experiments of [⁶⁴Cu]Cu-FS-594 to live PC3 cells, overexpressing human PD-L1 (sixfold);
- Fig. 2: shows a diagram as obtained in saturation binding experiments of [⁶⁴Cu]Cu-FS-594 to xenograft tissue sections, from PC3 cells overexpressing human PD-L1 (sixfold);
- Fig. 3: shows a diagram as obtained in saturation binding experiments of [⁶⁴Cu]Cu-FS-594 to live PC3 cells, lacking human PD-L1 expression;
- Fig. 4: shows PET images 1,5 h, 4,5 h, 24,5 h and 49 h after injection of [⁶⁴Cu]Cu-FS-594 in PC3 PD-L1 overexpressing and PC3 target-negative xenografts and
- Fig. 5: shows PET images 1,5 h, 7 h, 24,5 h and 49 h after injection of [⁶⁴Cu]Cu-FS-594 in MDA-MB-231 xenografts with natural PD-L1 expression.

### Examples

### General Procedures and Analytical Chemistry

All solvents and starting chemicals were obtained from commercial sources (abcr GmbH, Acros Organics, Alfa Aesar, BLDpharm, Merck, Carl Roth GmbH + Co. KG, Sigma-Aldrich, TCI GmbH, Thermo Fisher Scientific, VWR) and used without further purification. Any water used for reactions and purifications was ultra-pure water previously filtered through a Milli-Q^{®} IQ 7005 water purification system. The yields given are calculated from the chromatographically purified products characterized with NMR spectroscopy or LC-MS without consideration of the final purity. The compounds were named according to IUPAC nomenclature using ChemDraw Version 23.0.1.

### HPLC

Knauer Smartline system (Knauer Wissenschaftliche Geräte GmbH, Berlin, Germany) equipped with a Smartline pump 1000, the degasser system Smartline manager 5000 with performance at room temperature, UV-Vis-NIR Smartline detector 2500 for wavelength detection at 220 nm and 254 nm and a C18 column from Phenomenex (Gemini^{®} , 5 µm, 110 Å, LC Column 250x10 mm, System A OR Synergi^{™} , 4 µm, 80 Å, Hydro-RP Column 250x10 mm, System B). The purification was performed with the following conditions: 5-95% MeCN/H₂O, 5 min isocratic, then linear gradient in 25 minutes, then 5 min isocratic (Method 1) or without isocratic hold at beginning (Method 2), with constant 0.1% v/v TFA additive and a flow rate of 6.00 mL/min.

### NMR

All NMR spectra were recorded at 25 °C using a Bruker Avance III 400 MHz/Agilent DD2-400 MHz (¹H: 400 MHz, ¹³C: 101 MHz, ¹⁹F: 376 MHz) ¹³C-NMR spectra were recorded decoupled from ¹H-NMR spectra. ¹H NMR spectra were integrated to first order and analyzed using the Mestrenova software. The NMR spectra were calibrated to the signal of the solvent used. Chemical shifts δ are given in ppm, and coupling constants J are given in Hz. The following abbreviations were used to describe multiplicity: s = singlet, d = doublet, t = triplet, q = quartet, qu = quintet, m = multiplet.

### HRMS

High resolution mass spectra were obtained on a Q-TOF MS using electrospray ionization: Agilent 1260 Infinity II HPLC (Santa Clara, California, USA; pump G7104C, autosampler G7129C, column oven G7116A, DAD detector G7117C) coupled to γ detector Gabi Star (Raytest Isotopenmeßgeräte GmbH, Straubenhardt, Germany) followed by accurate mass Revident Q-TOF LC/Q-TOF G6575A. Unless otherwise stated, the measurements were performed in bypass mode using an eluent consisting of (A): MeCN and (B): 0.1% formic acid in H₂O; flow rate 0.2 mL/min). A reference mass solution containing hexakis(1H,1H,3H-tetrafluoropropoxy)phosphazene, and purine was continuously co-injected via dual AJS ESI source. The system was operated using Agilent Masshunter Workstation 3.6 - LC/MS data aquisition software (Version 12.0) and data evaluation was performed using Agilent Masshunter Workstation 3.6 Qualitative Analysis software (Version 12.0 Update 1).

### LC-MS

Mass spectrometry (MS) with electrospray ionization (ESI) was measured using a low resolution Xevo TQ-S mass spectrometer by Waters Corporation. Comparative values were calculated (calcd.) using ChemDraw Version 23.0.1.

### Thin layer chromatography

Thin layer chromatography was performed to monitor the progress of the respective reactions using Roth ROTI^{®} ChromaPlate Alu 60 UV TLC plates. The chromatograms were evaluated under 254 nm and 366 nm UV irradiation.

### Flash column chromatography

Crude products were purified via flash column chromatography at the Biotage^{®} Selekt System using BÜCHI FlashPure EcoFlex chromatography cartridges for normal phase and Biotage^{®} Sfär C18 Duo cartridges for reversed phase chromatography. The products were collected by tracking the UV activity in the range of 200 to 400 nm and/or by tracking evaporative light-scattering via the coupled Biotage^{®} Selekt ELSD-A120 detector. If not stated otherwise, the crude products were dissolved in suitable solvents (dichloromethane or ethyl acetate), silica was added, and the mixture was evaporated under reduced pressure and the silica adsorbed crude product was dry loaded via a pre-column.

### Radiolabeling

Copper-64 was produced by the nuclear reaction ⁶⁴Ni(p,n)⁶⁴Cu at the TR-Flex cyclotron (Advanced Cyclotron Systems Inc., ACSI, Canada) at the Helmholtz-Zentrum Dresden-Rossendorf. The radionuclide was obtained in a solution of 0.1-0.01 M HCl, and radiolabeling was carried out in a volume of approximately 200 µl in a 1 M HEPES solution, which was adjusted to pH 4.5 with 1 M HCl.

### Preparation of the starting compounds

### (5-(Methylsulfonyl)pyridine-3-yl)methanol (1)

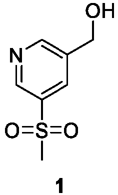

In a 250-mL Schlenk flask, (5-bromopyridin-3-yl)methanol (5.00 g, 26.6 mmol, 1.00 eq.) was dissolved in dimethyl sulfoxide (DMSO, 53 mL). After degassing the solution with argon for 30 min, copper(I) iodide (506 mg, 2.66 mmol, 0.10 eq.), sodium hydroxide (213 mg, 5.32 mmol, 0.20 eq.), sodium methylsulfinate (4.07 g, 39.9 mmol, 1.50 eq.), and (*R*)-proline (612 mg, 5.32 mmol, 0.20 eq.) were added countercurrently in one go. The flask was sealed, heated to 90 °C, and the reaction stirred at this temperature for 65 h. The solvent was then removed at 80 °C under high vacuum, and the residue was transferred to a separatory funnel with water and EtOAc. The phases were separated, and the aqueous phase was extracted 10 times with EtOAc. The combined organic phases were dried over Na₂SO₄, filtered, and concentrated under reduced pressure. The crude product was purified by column chromatography on SiO₂ (eluent EtOAc:MeOH 10:1, Rf=0.3). [5-(Methylsulfonyl)pyridin-3-yl)methanol (**1**, 3.44 g, 18.4 mmol, **69%**) was obtained as a pale yellow solid after removal of the eluent under vacuum.

¹H NMR (400 MHz, DMSO) δ 9.01 (s, 1H), 8.88 (s, 1H), 8.25 (s, 1H), 5.58 (t, J = 5.8 Hz, 1H), 4.67 (d, J = 5.8 Hz, 2H), 3.33 (s, 3H). ¹³C NMR (101 MHz, DMSO) δ 152.34, 146.14, 138.97, 132.75, 60.01, 43.70.

### 5-Chloro-2,4-dihydroxybenzaldehyde (2)

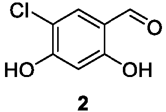

2,4-Dihydroxybenzaldehyde (15.0 g, 109 mmol, 1.00 eq.) was placed in a 1 L three-necked flask fitted with a reflux condenser and dissolved in CH₃Cl (600 mL). The solution was heated to 60 °C, and *N*-chlorosuccinimide (15.2 g, 114 mmol, 1.05 eq.) was added. After 17 h, 0.6 mL of concentrated HCl was added, and the solution was stirred at 60 °C for a further 6 h. The reaction mixture was then cooled to room temperature, transferred to a separatory funnel, and washed successively with 100 mL of water and 100 mL of saturated NaCl solution. The combined aqueous phases were extracted three times with EtOAc, and the combined organic phases were subsequently dried over Na₂SO₄. The mixture was filtered, the solvent was concentrated under reduced pressure, and the crude product was adsorbed onto silica gel and purified by column chromatography on SiO2 (eluent cyclohexane:EtOAc 7:1, R_{f}=0.26). 5-Chloro-2,4-dihydroxybenzaldehyde (**2**, 13.9 g, 80.6 mmol, **74%**) was obtained as a white solid after removal of the eluent *in vacuo.*

¹H NMR (400 MHz, DMSO) δ 11.38 (s, 1H), 10.86 (s, 1H), 9.97 (s, 1H), 7.59 (s, 1H), 6.58 (s, 1H). ¹³C NMR (101 MHz, DMSO) δ 188.91, 161.37, 159.97, 130.33, 116.05, 112.26, 103.55.

### 5-Chloro-2-hydroxy-4-((2-methoxyethoxy)methoxy)benzaldehyde (3)

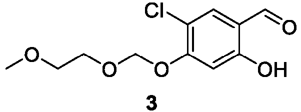

A 500 mL Schlenk flask was alternately heated under high vacuum (10⁻³ mbar) and flushed with argon three times. Compound 2 (10.0 g, 58.0 mmol, 1.00 eq.) was placed in it and dissolved in absolute tetrahydrofuran (THF, 220 mL). Diisopropylethylamine (DIPEA, 20.2 mL, 15.0 g, 116 mmol, 2.00 eq.) was added, and the solution was cooled to 0 °C. 2-Methoxyethoxymethyl chloride (8.00 mL, 8.66 g, 69.5 mmol, 1.20 eq.) was added, and the reaction mixture was slowly warmed to room temperature and stirred for a total of 16 h. The mixture was then transferred to a separatory funnel with water and EtOAc, shaken, and the phases were separated. The aqueous phase was extracted three times with EtOAc. The combined organic phases were dried over Na₂SO₄, filtered, and *concentrated in vacuo*. The crude product was adsorbed onto silica gel and purified by column chromatography on SiO₂ using a Biotage Select system (eluent: EtOAc in petroleum ether, 0% to 100% gradient). After removal of the solvent *in vacuo,* 5-chloro-2-hydroxy-4-((2-methoxyethoxy)methoxy)benzaldehyde (**3**, 12.1 g, 46.3 mmol, **80%**) was obtained as a yellow solid.

R_{f}=0.35 (Cyclohexane:EtOAc 4:1), ¹H NMR (400 MHz, CDCl₃) δ 11.26 (s, 1H), 9.69 (s, 1H), 7.51 (s, 1H), 6.78 (s, 1H), 5.39 (s, 2H), 3.88 - 3.81 (m, 2H), 3.59 - 3.52 (m, 2H), 3.37 (s, 3H). ¹³C NMR (101 MHz, CDCl₃) δ 194.00, 162.71, 159.62, 134.19, 115.85, 114.93, 103.81, 94.04, 71.49, 68.79, 59.17.

### 5-Chloro-4-((2-methoxyethoxy)methoxy)-2-((5-(methylsulfonyl)pyridin-3-yl)methoxy)benzaldehyde (4)

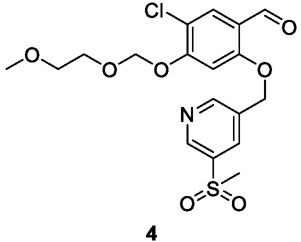

Compound **1** (2.50 g, 13.4 mmol, 1.00 eq.) was placed in a 100 mL round-bottomed flask and dissolved in SOCl₂ (25 mL). The reaction mixture was stirred at room temperature for 3.5 h, and the solvent was then removed under reduced pressure. The resulting intermediate was placed under argon (evacuation three times followed by flooding with protective gas) in the same flask without further workup and dissolved in *N,N*-dimethylformamide (DMF, 50 mL). Compound 3 (3.48 g, 13.4 mmol, 1.00 eq.), Cs₂CO₃ (13.1 g, 40.1 mmol, 3.00 eq.), and KI (2.22 g, 13.4 mmol, 1.00 eq.) were added sequentially, and the suspension was stirred at room temperature for 3 h. The mixture was transferred to a separatory funnel with water and EtOAc, shaken, and the phases were separated. The aqueous phase was extracted four times with EtOAc, and the combined organic phases were dried over Na₂SO₄, filtered, and concentrated *in vacuo.* The crude product was adsorbed onto silica gel and purified by column chromatography on SiO₂ using a Biotage Select system (eluent: EtOAc in cyclohexane, 0% to 100% gradient). After removal of the solvent *in vacuo,* 5-chloro-4-((2-methoxyethoxy)methoxy)-2-((5-(methylsulfonyl)pyridin-3-yl)methoxy)benzaldehyde (**4**, 5.33 g, 12.4 mmol, **93%**) was obtained as an as a white solid.

R_{f}=0.24 (EtOAc), ¹H NMR (400 MHz, CDCl₃) δ 10.27 (s, 1H), 9.16 (d, J = 2.2 Hz, 1H), 8.99 (d, J = 2.1 Hz, 1H), 8.36 (dd, J = 2.2, 2.1 Hz, 1H), 7.86 (s, 1H), 7.01 (s, 1H), 5.42 (s, 2H), 5.27 (s, 2H), 3.92 - 3.85 (m, 2H), 3.61 - 3.54 (m, 2H), 3.37 (s, 3H), 3.16 (s, 3H). ¹³C NMR (101 MHz, CDCl₃) δ 186.69, 159.98, 158.92, 153.37, 148.63, 137.36, 134.45, 132.39, 130.67, 120.24, 117.44, 101.03, 94.33, 71.46, 68.47, 67.85, 59.12, 44.94.

### 5-Chloro-4-hydroxy-2-((5-(methylsulfonyl)pyridin-3-yl)methoxy)benzaldehyde (5)

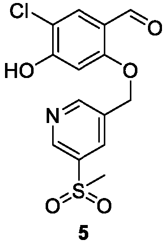

In a 500 mL round-bottomed flask, Compound **4** (7.00 g, 16.3 mmol, 1.00 eq.) was placed in air and dissolved in CH₂Cl₂ (175 mL) and trifluoroacetic acid (TFA, 175 mL). The mixture was stirred for 21 h at room temperature, the solvent was concentrated *in vacuo* to a few milliliters, and the mixture was layered with cyclohexane. 5-chloro-4-hydroxy-2-((5-(methylsulfonyl)pyridin-3-yl)methoxy)benzaldehyde (**5**) was precipitated by addition of EtOAc, and stirring was continued for a further 1 h. The suspension was transferred to a centrifuge tube and centrifuged at 10,000 rpm for 15 min. The supernatant was removed, and the residue was slurried with EtOAc. The mixture was centrifuged again, and the procedure was repeated two more times. The precipitate was transferred to a flask and dried under vacuum (10⁻³ mbar). The product **5** (5.56 g, 16.3 mmol, **quant.**) was obtained as a gray solid.

¹H NMR (400 MHz, DMSO) δ 11.59 (s, 1H), 10.15 (s, 1H), 9.09 (s, 1H), 9.07 (s, 1H), 8.47 (s, 1H), 7.67 (s, 1H), 6.81 (s, 1H), 5.38 (s, 2H), 3.37 (s, 3H). ¹³C NMR (101 MHz, DMSO) δ 186.45, 160.34, 159.91, 153.24, 147.40, 136.97, 134.24, 132.74, 129.51, 117.86, 113.53, 101.49, 67.12, 43.63.

### Ethyl 2-bromo-3-iodobenzoate (6)

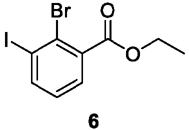

A 50 mL Schlenk flask was alternately evaporated three times under high vacuum (10⁻³ mbar) and flushed with argon. 2-Bromo-3-iodobenzoic acid (4.00 g, 12.2 mmol, 1.00 eq.) and K₂CO₃ (4.99 g, 36.1 mmol, 2.95 eq.) were placed in it, and the flask was again evacuated and flushed with argon. The substances were suspended in DMF, and EtI (1.48 mL, 2.86 g, 18.4 mmol, 1.50 eq.) was added at room temperature. The mixture was warmed to 40 °C and stirred at this temperature for 8 h. The reaction was then quenched by adding 5 mL of saturated NH₄Cl solution, and the mixture was transferred to a separatory funnel with EtOAc. The phases were separated, and the aqueous phase was extracted three times with EtOAc. The combined organic phases were washed with NaCl solution, dried over Na₂SO₄, filtered, and the solvent was removed *in vacuo.* Ethyl 2-bromo-3-iodobenzoate (**6**) was obtained as a light yellow oil and used without further purification.

¹H NMR (400 MHz, CDCl₃) δ 7.98 (dd, J = 7.9, 1.6 Hz, 1H), 7.56 (dd, J = 7.7, 1.6 Hz, 1H), 7.06 (dd, J = 7.9, 7.7 Hz, 1H), 4.40 (q, J = 7.1 Hz, 2H), 1.40 (t, J = 7.1 Hz, 3H).

### (2-Bromo-3-iodophenyl)methanol (7)

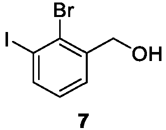

Compound **6** (from the previous reaction, 1.00 eq.) was added to a 500 mL round-bottomed flask and placed under argon (evacuation three times followed by flooding with inert gas). The starting material was dissolved in absolute CH₂Cl₂ (41 mL) and cooled to 0 °C. Diisobutylaluminum hydride (1 M in hexane, 36.7 mL, 36.7 mmol, 3.00 eq.) was added dropwise over a period of 10 min, and the solution was stirred for a further hour at 0 °C. 2-Propanol (10 mL) and water (15 mL) were carefully added successively. The mixture was acidified with concentrated HCl until all substances had completely dissolved. The solution was transferred to a separatory funnel with EtOAc, and the phases were separated. The aqueous phase was extracted three times with EtOAc, and the combined organic phases were then dried over Na₂SO₄, filtered, and the solvent was evaporated under reduced pressure. The crude product was adsorbed onto silica gel and purified by column chromatography on SiO₂ using a Biotage Select system (eluent: EtOAc in cyclohexane, 0% to 20% gradient). After removal of the solvent *in vacuo,* (2-bromo-3-iodophenyl)methanol (**7**, 3.65 g, 11.6 mmol, **95% over 2 steps**) was obtained as a white solid.

R_{f}=0.27 (Cyclohexane:EtOAc 4:1), ¹H NMR (400 MHz, CDCl₃) δ 7.82 (d, J = 7.8 Hz, 1H), 7.47 (d, J = 7.5 Hz, 1H), 7.06 (dd, J = 7.8, 7.5 Hz, 1H), 4.75 (s, 2H). ¹³C NMR (101 MHz, CDCl₃) δ 142.30, 139.52, 129.08, 128.97, 128.04, 103.08, 66.95.

### (2-Bromo-[1,1'-biphenyl]-3-yl)methanol (8)

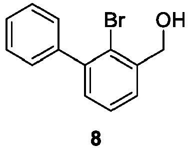

Compound **7** (1.70 g, 5.43 mmol, 1.00 eq.), phenylboronic acid (696 mg, 5.70 mmol, 1.05 eq.), K₂CO₃ (1.50 g, 10.9 mmol, 2.00 eq.), and tris(2-methylphenyl)phosphane (66.1 mg, 217 µmol, 4 mol%) were placed in a 100 mL Schlenk flask and dissolved in water (10 mL) and toluene (40 mL). The two phases were degassed with argon for 45 min, after which Pd₂(dba)₃ (49.8 mg, 54.3 µmol, 1 mol%) was added in countercurrent argon. The sealed flask was heated to 90 °C, which rapidly decolorized the initially red solution. The mixture was stirred at this temperature for 16 h before cooling to room temperature and filtering through Celite^{®} 545 with EtOAc. The phases were separated in a separatory funnel. The organic phase was washed with saturated NaHCO₃ solution and saturated NaCl solution. The combined aqueous phases were extracted three times with EtOAc, then dried over Na₂SO₄, filtered, and the solvent was evaporated under reduced pressure. The crude product was adsorbed onto silica gel and purified by column chromatography on SiO₂ using a Biotage Select system (eluent: EtOAc in cyclohexane, 0% to 50% gradient). After removal of the solvent *in vacuo,* (2-bromo-[1,1'-biphenyl]-3-yl)methanol (**8**, 1.28 g, 4.86 mmol, **90%**) was obtained as a slightly yellowish, slightly impure oil.

R_{f}=0.21 (EtOAc:Cyclohexane 1:4), ¹H NMR (400 MHz, CDCl₃) δ 7.51 - 7.48 (m, 1H), 7.47 - 7.34 (m, 6H), 7.29 - 7.24 (m, 1H), 4.83 (d, J = 6.4 Hz, 2H), 2.14 (t, J = 6.4 Hz, 1H). ¹³C NMR (101 MHz, CDCl₃) δ 143.61, 141.49, 140.70, 130.58, 129.58, 128.11, 127.88, 127.75, 127.47, 123.07, 66.05.

### 2-Bromo-3-(bromomethyl)-1,1'-biphenyl (9)

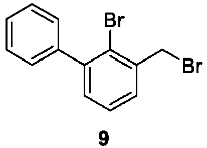

A 50 mL round-bottomed flask was alternately heated under high vacuum (10⁻³ mbar) and flushed with argon three times. Compound **8** (1.21 g, 4.59 mmol, 1.00 eq.) was dissolved in absolute CH₂Cl₂ (20 mL) and cooled to 0 °C. PBr₃ (523 µL, 1.49 g, 5.50 mmol, 1.20 eq.) was slowly added dropwise, and the solution was stirred for 4 h at 0 °C. Silica gel was added, and the solvent was removed under vacuum. The crude product was purified by column chromatography on SiO₂ using a Biotage Select system (eluent: cyclohexane). After removal of the solvent *in vacuo,* 2-bromo-3-(bromomethyl)-1,1'-biphenyl (**9**, 1.28 g, 3.94 mmol, **86%**) was obtained as a colorless oil.

R_{f}=0.26 (Cyclohexane), ¹H NMR (400 MHz, CDCl₃) δ 7.51 - 7.31 (m, 7H), 7.28 - 7.24 (m, 1H), 4.72 (s, 2H). ¹³C NMR (101 MHz, CDCl₃) δ 144.49, 141.49, 137.99, 131.51, 131.47, 130.35, 129.51, 128.14, 127.87, 127.58, 127.54, 125.11, 34.85.

### 4-((2-Bromo-[1,1'-biphenyl]-3-yl)methoxy)-5-chloro-2-((5-(methylsulfonyl)pyridin-3-yl)methoxy)benzaldehyde (10)

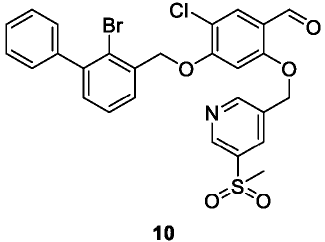

A 50 ml round-bottomed flask was alternately heated under high vacuum (10⁻³ mbar) and flushed with argon three times. DMF (20 ml) was added, followed by the addition of Compound **5** (560 mg, 1.64 mmol, 1.00 eq.), Compound 9 (534 mg, 1.64 mmol, 1.00 eq.), and NaHCO₃ (413 mg, 4.92 mmol, 3.00 eq.) in countercurrent argon. The suspension was stirred at room temperature for 15 h, the solvent then evaporated under reduced pressure and the residue adsorbed onto silica gel. The crude product was purified by column chromatography on SiO₂ using a Biotage Select system (eluent: n-hexane:THF 4:6 with 1% NEt₃). After removal of the solvent *in vacuo,* 4-((2-bromo-[1,1'-biphenyl]-3-yl)methoxy)-5-chloro-2-((5-(methylsulfonyl)pyridin-3-yl)methoxy)benzaldehyde (**10**, 793 mg, 1.35 mmol, **82%**) was obtained as a beige solid.

R_{f}=0.34 (Cyclohexane:THF 4:6 with 1% Et₃N), ¹H NMR (400 MHz, DMSO) δ 10.23 (s, 1H), 9.10 (s, 1H), 9.09 (s, 1H), 8.52 (t, J = 1.9 Hz, 1H), 7.76 (s, 1H), 7.70 (dd, J = 7.7, 1.7 Hz, 1H), 7.54 (t, J = 7.6 Hz, 1H), 7.51 - 7.38 (m, 6H), 7.29 (s, 1H), 5.57 (s, 2H), 5.46 (s, 2H), 3.37 (s, 3H). ¹³C NMR (101 MHz, DMSO) δ 186.81, 160.72, 159.28, 153.20, 147.46, 143.16, 140.71, 137.02, 135.63, 134.32, 132.68, 131.46, 129.37, 129.22, 128.92, 128.16, 127.87, 127.75, 123.23, 118.94, 114.91, 100.49, 71.53, 67.76, 43.61.

### tert-Butyl-(2-(2-(2-aminoethoxy)ethoxy)ethyl)carbamate (11)

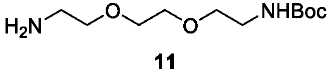

A 250 mL round-bottomed flask was alternately heated under high vacuum (10⁻³ mbar) and flushed with argon three times. Absolute CH₂Cl₂ (40 ml) was added, 2,2'-(ethylenedioxy)diethylamine (8.15 g, 55.0 mmol, 6.00 eq.) was dissolved in it, and the mixture was cooled to 0 °C. Separately, di-tert-butyl dicarbonate (2.00 g, 9.16 mmol, 1.00 eq.) was dissolved in absolute CH₂Cl₂ (20 ml) in a 50 ml conical-bottomed flask and then added dropwise to the first solution over 4 h using a syringe pump. The solution was stirred for a further 17 h, slowly warming to room temperature. The solvent was removed under vacuum, and the residue was taken up in water and transferred to a separatory funnel. The solution was washed five times with CH₂Cl₂. The combined organic phases were dried over MgSO₄, filtered, and the solvent was removed under reduced pressure. *tert*-Butyl (2-(2-(2-aminoethoxy)ethoxy)ethyl)carbamate (**11**, 2.28 g, 9.16 mmol, **quant**.) was obtained as a colorless oil and used without further workup.

¹H NMR (600 MHz, CDCl₃) δ 5.13 (s, 1H), 3.62 (s, 4H), 3.59 - 3.51 (m, 4H), 3.34 - 3.29 (m, 2H), 2.90 (t, J = 5.2 Hz, 2H), 1.44 (s, 9H).

### (9H-Fluoren-9-yl)methyl (2,2-dimethyl-4-oxo-3,8,11-trioxa-5-azatridecan-13-yl)carbamate (12)

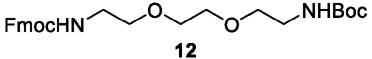

Compound **11** (2.19 g, 8.82 mmol, 1.00 eq.) was placed in a 100 mL round-bottomed flask, placed under argon (evacuation three times followed by purging with inert gas), and dissolved in CH₂Cl₂ (25 mL). Et₃N (1.35 mL, 983 mg, 9.71 mmol, 1.10 eq.) was added, and the solution was cooled to 0 °C. Fluorenylmethyloxycarbonyl chloride (2.74 g, 10.6 mmol, 1.20 eq.) was added under argon countercurrent, and the solution was stirred at 0 °C for 2 h. The solvent was then removed *in vacuo,* the residue was adsorbed onto silica gel, and purified by column chromatography on SiO₂ using a Biotage Select system (eluent: EtOAc in cyclohexane, 0% to 50% gradient). After removal of the eluent *in vacuo,* (9H-fluoren-9-yl)methyl (2,2-dimethyl-4-oxo-3,8,11-trioxa-5-azatridecan-13-yl)carbamate (**12,** 4.15 g, 8.82 mmol, **quant**.) was obtained as a colorless oil.

¹H NMR (400 MHz, CDCl₃) δ 7.76 (d, J = 7.5 Hz, 2H), 7.60 (d, J = 7.5 Hz, 2H), 7.40 (ddd, J = 7.4, 7.4, 1.2 Hz, 2H), 7.31 (ddd, J = 7.4, 7.4, 1.2 Hz, 2H), 5.33 (s, 1H), 5.00 (s, 1H), 4.41 (d, J = 7.0 Hz, 2H), 4.23 (t, J = 7.0 Hz, 1H), 3.64 - 3.53 (m, 8H), 3.41 (q, J = 5.3 Hz, 2H), 3.31 (d, J = 5.0 Hz, 2H), 1.43 (s, 9H).

### (9H-Fluoren-9-yl)methyl-(2-(2-(2-aminoethoxy)ethoxy)ethyl)carbamate (13)

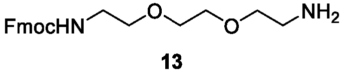

Compound **12** (4.27 g, 9.08 mmol, 1.00 eq.) was dissolved in CH₂Cl₂ in a 100 mL round-bottomed flask. TFA was slowly added dropwise, and the solution was stirred for 2 h at room temperature. The solvent was then removed *in vacuo,* and the residue was adsorbed onto silica gel. The crude product was purified by column chromatography on SiO₂ using a Biotage Select system (eluent: MeOH in CH₂Cl₂, 0% to 80% gradient). After removal of the solvent *in vacuo,* (9H-fluoren-9-yl)methyl-(2-(2-(2-aminoethoxy)ethoxy)ethyl)carbamate (**13**, 3.36 g, 9.08 mmol, **quant.**) was obtained as a slightly yellow, sticky solid.

¹H NMR (400 MHz, CDCl₃) δ 7.73 (d, J = 7.5 Hz, 2H), 7.57 (d, J = 7.7 Hz, 2H), 7.41 - 7.33 (m, 2H), 7.32 - 7.25 (m, 2H), 5.64 (s, 1H), 4.38 (d, J = 7.2 Hz, 2H), 4.18 (t, J = 6.7 Hz, 1H), 3.63 (t, J = 5.0 Hz, 2H), 3.57 - 3.43 (m, 6H), 3.33 - 3.28 (m, 2H), 3.07 (t, J = 4.9 Hz, 2H). ¹³C NMR (101 MHz, CDCl₃) δ 156.96, 144.06, 141.43, 127.83, 127.21, 125.18, 120.06, 70.21, 70.11, 66.85, 66.72, 47.30, 40.85, 39.61.

### (9H-Fluoren-9-yl)methyl-(2-(2-(2-(((4-((2-bromo-[1,1'-biphenyl]-3-yl)methoxy)-5-chloro-2-((5-(methylsulfonyl)pyridin-3-yl)methoxy) benzyl)amino)ethoxy)ethoxy)ethyl)carbamate (14)

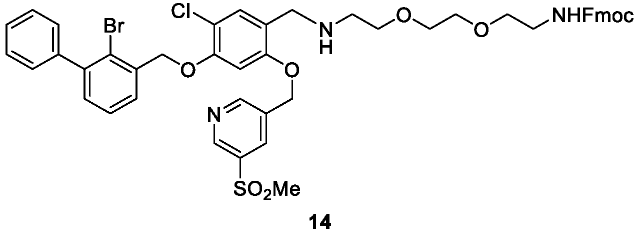

ZnCl₂ (81.8 mg, 600 µmol, 3.00 eq.) and Compound 10 (117 mg, 200 µmol, 1.00 eq.) were placed in a 10 mL round-bottomed flask and suspended in DMF (1.25 mL). **13** (222 mg, 600 µmol, 3.00 eq.), dissolved in MeOH (1.25 mL), was added and stirred for 1 hour at room temperature. The mixture was then cooled to 0 °C, and NaBH₃CN (18.9 mg, 300 µmol, 1.5 eq.) was added. The suspension was stirred for 21 h, slowly warming to room temperature. At the end of the reaction time, a clear yellow solution was obtained. This solution was transferred to a separatory funnel with EtOAc and THF and washed with NaCl solution. The phases were separated, and the aqueous phase was extracted three more times with EtOAc/THF (9:1). The combined organic phases were dried over Na₂SO₄, filtered, and the solvent was removed *in vacuo.* The crude product was adsorbed onto silica gel and roughly purified by column chromatography on SiO₂ using a Biotage Select system (eluent: THF in cyclohexane, 0% to 100% gradient with 1% Et₃N). The product was subsequently obtained as an impure yellowish oil, and was used without further purification.

R_{f}=0.96 (Cyclohexane:THF 4:6 with 1 % Et₃N)

### 2-(2-(2-Aminoethoxy)ethoxy)-N-(4-((2-bromo-[1,1'-biphenyl]-3-yl)methoxy)-5-chloro-2-((5-(methylsulfonyl)pyridin-3-yl)methoxy)benzyl)ethane-1-amine (15)

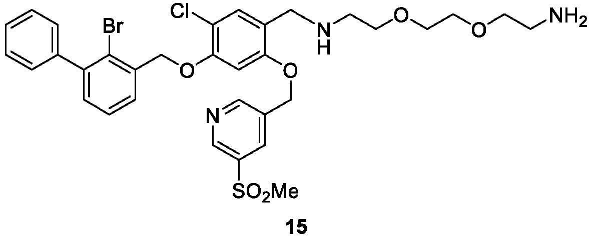

Compound **14** (from the previous reaction, 1.00 eq) was dissolved in DMF (2.0 mL) in a 50 mL round-bottomed flask. Piperidine (200 µL) was added, and the solution was stirred at room temperature for 1.5 h. The solvent was then removed *in vacuo,* and the residue was dissolved in a mixture of water and acetonitrile (1:1). The crude product was purified by column chromatography on C-18-functionalized SiO₂ (Biotage^{®} Sfär C18 Duo 100 Å 30 µm column) using a Biotage Select system (eluent: acetonitrile in water, 0% to 100% gradient with 0.1% TFA), then on SiO₂ (eluent: MeOH in CH₂Cl₂, 0% to 50% gradient with 1% Et₃N) and finally again on C-18-functionalized SiO₂ (eluent: acetonitrile in water, 0% to 100% gradient with 0.1% formic acid). After removal of the solvent *in vacuo,* 2-(2-(2-aminoethoxy)ethoxy)-N-(4-((2-bromo-[1,1'-biphenyl]-3-yl)methoxy)-5-chloro-2-((5-(methylsulfonyl)pyridin-3-yl)methoxy)benzyl)ethan-1-amine (**15**, 63.0 mg, 87.6 µmol, **44% over 2 steps**) was obtained as a white solid.

¹H NMR (400 MHz, DMSO) δ 9.13 (d, J = 2.0 Hz, 1H), 9.09 (d, J = 2.2 Hz, 1H), 8.62 - 8.56 (m, 1H), 7.71 (s, 1H), 7.68 (dd, J = 7.6, 1.7 Hz, 1H), 7.53 (t, J = 7.6 Hz, 1H), 7.51 - 7.37 (m, 6H), 7.20 (s, 1H), 5.46 (s, 2H), 5.37 (s, 2H), 4.12 (dd, J = 5.1, 5.1 Hz, 2H), 3.73 (t, J = 5.4 Hz, 2H), 3.61 (t, J = 5.3 Hz, 2H), 3.55 (s, 4H), 3.38 (s, 3H), 3.10 - 3.02 (m, 2H), 2.97 - 2.90 (m, 2H). ¹³C NMR (101 MHz, DMSO) δ 156.42, 155.00, 153.37, 147.25, 143.11, 140.76, 136.97, 136.12, 134.73, 133.04, 132.77, 131.28, 129.22, 129.18, 128.17, 127.81, 127.75, 123.11, 113.67, 113.05, 100.14, 71.16, 69.52, 69.48, 67.34, 66.54, 65.57, 45.82, 43.96, 43.65, 38.42. Exact mass calculated for [M + H]+: m/z = 718.1348; measured: m/z = 718.1353.

### (((((9H-Fluoren-9-yl)methoxy)carbonyl)(sulfo)-D-alanyl)(sulfo)-D-alanyl)(sulfo)-D-alanylglycine (16)

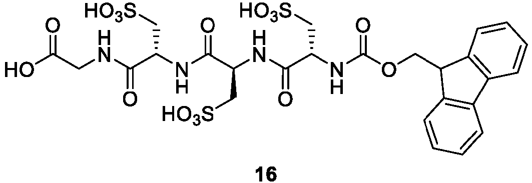

In a 60 mL reactor for peptide synthesis, 2-chlorotrityl chloride resin (3.75 g, 6.00 mmol, 2.00 eq.) was first weighed, suspended in CH₂Cl₂ (50 mL), and shaken for 30 min. The solvent was filtered off. Fmoc-Gly-OH (892 mg, 3.00 mmol, 1.00 eq.), dissolved in approximately 45 mL of CH₂Cl₂, and DIPEA (1.05 mL, 6.00 mmol, 2.00 eq.) were then added to the swollen resin. The reactor was sealed and shaken for 2 h at room temperature. The reaction solution was filtered off, and the resin was washed three times with CH₂Cl₂. To endcap all unreacted reactive groups, the resin was treated three times with a mixture of CH₂Cl₂, MeOH, and DIPEA (17:1:2, 45 mL), each time shaken for 2 min. The solution was filtered, and the resin was washed three times with CH₂Cl₂ and then three times with Et₂O (45 mL each). For Fmoc deprotection, the resin was then swollen in DMF (45 mL), filtered, and treated with piperidine (20% in DMF, 45 mL). The resin was shaken for 15 min, filtered, and the process repeated one more time. The resin was then washed three times with DMF (45 mL). For conjugation of the additional amino acids, the resin was first suspended in DMF (15 mL) and Fmoc-L-cysteic acid (2.35 g, 6.00 mmol, 2.00 eq.) dissolved in DMF (15 mL), DIPEA (2.05 mL, 12.0 mmol, 4.00 eq.), and HATU (2.28 g, 6.00 mmol, 2.00 eq.) dissolved in DMF (15 mL) were added quickly in succession. The sealed reactor was shaken for 1.5 h at room temperature, the reaction mixture was then filtered off, and the resin was washed three times with DMF (45 mL). For Fmoc deprotection, the resin was again treated twice with piperidine (20% in DMF, 45 mL) and shaken for 15 min each time. The process was repeated three times in total, with no Fmoc cleavage occurring the last time. Instead, the resin was washed three times each with DMF (45 mL), CH₂Cl₂ (45 mL), and finally with Et₂O (45 mL). To cleave the linker **16**, the resin was treated with hexafluoroisopropanol (20% in CH₂Cl₂, 45 mL) and shaken for 1 h. The residue was filtered off and the process was repeated. The resin was then washed three more times with DMF (45 mL). The combined solutions were concentrated *in vacuo.* The residue was purified by column chromatography on C-18-functionalized SiO₂ (Biotage^{®} Sfär C18 Duo 100 Å 30 µm column) using a Biotage Select system (eluent: acetonitrile in water, 0% to 100% gradient with 0.1% TFA). (((((9*H*-Fluoren-9-yl)methoxy)carbonyl)(sulfo)-*D*-alanyl)(sulfo)-*D*-alanyl)(sulfo)-*D*-alanylglycine (**16**, 1.07 g, 1.43 mmol, **48%**) was obtained as a yellowish solid.

Mass calculated for [M - H]⁻: m/z = 749.07; measured: m/z = 749.22.

### (16R,19R,22R)-22-((((9H-Fluoren-9-yl)methoxy)carbonyl)amino)-1-(((2-Bromo-[1,1'-biphenyl]-3-yl)methoxy)-5-chloro-2-((5-(methylsulfonyl)pyridin-3-yl)methoxy)phenyl)-12,15,18,21-tetraoxo-16,19-bis(sulfomethyl)-5,8-dioxa-2,11,14,17,20-pentaazatricosan-23-sulfonic acid (17)

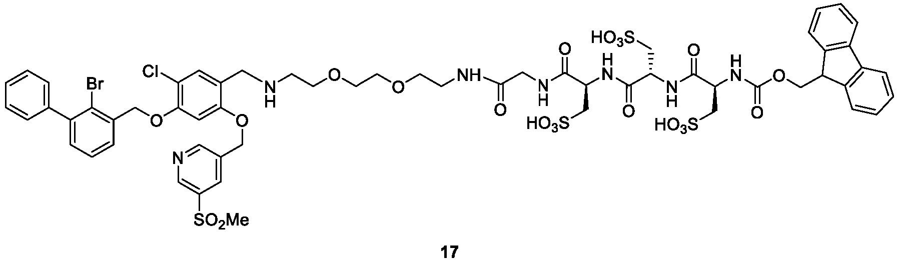

In a 10 mL round-bottomed flask, **16** (33.8 mg, 45.0 µmol, 3.00 eq.) was placed in DMF (300 µL). DIPEA (13.1 µL, 75.0 µmol, 5.00 eq.) and Compound **15** (10.8 mg, 15.0 µmol, 1.00 eq.) dissolved in DMF (150 µL) were added successively. The solution was cooled to 0 °C, and Oxyma Pure (6.40 mg, 45.0 µmol, 3.00 eq.) and 1-ethyl-3-(3-dimethylaminopropyl)carbodiimide (EDC, 6.99 mg, 45.0 µmol, 3.00 eq.) were added. The solution was stirred for 17 h while slowly warming to room temperature. The solvent was then evaporated under reduced pressure, and the residue was dissolved in a 1:1 mixture of water and MeCN. The crude product was purified by HPLC (System A). The still slightly impure intermediate (16*R*,19*R*,22*R*)-22-((((9*H*-fluoren-9-yl)methoxy)carbonyl)amino)-1-(4-((2-bromo-[1,1'-biphenyl]-3-yl)methoxy)-5-chloro-2-((5-(methylsulfonyl)pyridin-3-yl)methoxy)phenyl)-12,15,18,21-tetraoxo-16,19-bis(sulfomethyl)-5,8-dioxa-2,11,14,17,20-pentaazatricosan-23-sulfonic acid (**17**) was obtained as a white solid after removal of the solvent *in vacuo.*

Mass calculated for [M - H]-: m/z = 1448.19; measured: m/z = 1448.04.

### (16R,19R, 22R)-22-Amino-1-(4-((2-bromo-[1,1'-biphenyl]-3-yl)methoxy)-5-chloro-2-((5-(methylsulfonyl)pyridin-3-yl)methoxy)phenyl)-12,15,18,21-tetraoxo-16,19-bis(sulfomethyl)-5,8-dioxa-2,11,14,17,20-pentaazatricosan-23-sulfonic acid (18)

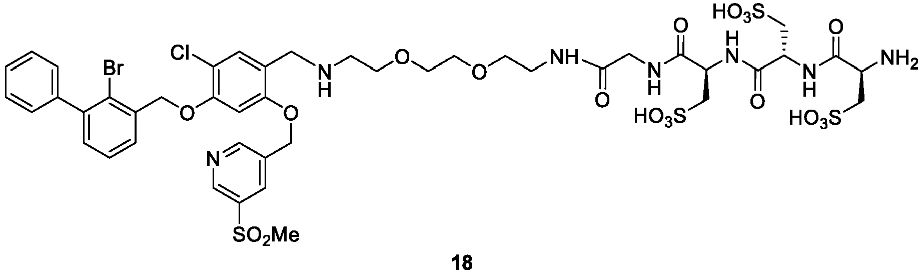

**17** (from the previous reaction, 1.00 eq.) was dissolved in DMF (500 µl) in a 25 mL round-bottomed flask. Piperidine (50 µl) was added, and the solution was stirred at room temperature for 1.5 h. The solvent was then evaporated *in vacuo,* and the residue was taken up in a 1:1 mixture of water and MeCN. The crude product was purified by column chromatography on C-18-functionalized SiO₂ (Biotage^{®} Sfär C18 Duo 100 Å 30 µm column) using a Biotage Select system (eluent: acetonitrile in water, 0% to 100% gradient with 0.1% formic acid). After removal of the solvent, (16*R*,19*R*,22*R*)-22-amino-1-(4-((2-bromo-[1,1'-biphenyl]-3-yl)methoxy)-5-chloro-2-((5-(methylsulfonyl)pyridin-3-yl)methoxy)phenyl)-12,15,18,21-tetraoxo-16,19-bis(sulfomethyl)-5,8-dioxa-2,11,14,17,20-pentaazatricosane-23-sulfonic acid (**18**, 10.2 mg, 8.30 µmol, **55% over 2 steps**) was obtained as a white solid.

Mass calculated for [M - H]-: m/z = 1226.12; measured: m/z = 1226.02.

### (5-(Methylsulfonyl)pyridin-3-yl)methanol (21)

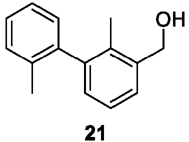

(3-Bromo-2-methylphenyl)methanol (7.04 g, 35.0 mmol, 1.00 eq.), o-tolyl-boronic acid (5.23 g, 38.5 mmol, 1.10 eq.), and K₂CO₃ (16.0 g, 116 mmol, 3.30 eq.) were placed in a 250 mL Schlenk flask and dissolved in water (64 mL) and 1,4-dioxane (95 mL). The two phases were degassed with argon for 1 h, after which Pd(dppf)Cl₂*CH₂Cl₂ (1.43 g, 1.75 mmol, 5 mol%) was added under argon countercurrent. The sealed flask was heated to 100 °C and stirred at this temperature for 16 h before cooling to room temperature and filtering through Celite^{®} 545 with EtOAc. The phases were separated in a separatory funnel. The organic phase was washed with saturated NaHCO₃ solution and saturated NaCl solution. The combined aqueous phases were extracted three times with EtOAc, then dried over Na₂SO₄, filtered, and the solvent was evaporated under reduced pressure. The crude product was adsorbed onto silica gel and purified by column chromatography on SiO₂ using a Biotage Select system (eluent: EtOAc in cyclohexane, 0% to 50% gradient). After removal of the solvent *in vacuo,* 5-(methylsulfonyl)pyridin-3-yl)methanol (**21**, 5.91 g, 27.83 mmol, **80%**) was obtained as a colorless solid.

R_{f}=0.18 (n-hexane:EtOAc 4:1); ¹H NMR (400 MHz, CDCl₃) δ 7.38 (d, J = 7.5 Hz, 1H), 7.30 - 7.17 (m, 3H), 7.12 - 7.05 (m, 2H), 4.77 (s, 2H), 2.05 (s, 4H), 2.04 (s, 3H). ¹³C NMR (101 MHz, CDCl₃) δ 142.47, 141.80, 139.10, 136.06, 134.12, 129.91, 129.54, 129.20, 127.33, 126.70, 125.70, 125.67, 64.16, 20.02, 15.44.

### 3-(Bromomethyl)-2,2'-dimethyl-1,1'-biphenyl (22)

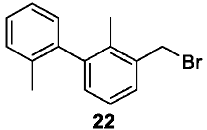

A 100 mL round-bottomed flask was alternately heated under high vacuum (10⁻³ mbar) and flushed with argon three times. Compound **21** (2.00 g, 9.42 mmol, 1.00 eq.) was dissolved in absolute CH₂Cl₂ (40 mL) and cooled to 0 °C. PBr₃ (1.07 mL, 3.06 g, 11.3 mmol, 1.20 eq.) was slowly added dropwise, and the solution was stirred at 0 °C for 4 h. Silica gel was added, and the solvent was removed under vacuum. The crude product was purified by column chromatography on SiO₂ using a Biotage Select system (eluent: EtOAc in petroleum ether, 0% to 20% gradient). After removal of the solvent *in vacuo,* 3-(bromomethyl)-2,2'-dimethyl-1,1'-biphenyl (**22**, 2.54 g, 9.23 mmol, 98%) was obtained as a colorless solid.

R_{f}=0.44 (Cyclohexane:EtOAc 4:1); ¹H NMR (400 MHz, CDCl₃) δ 7.33 (dd, J = 7.6, 1.5 Hz, 1H), 7.29 - 7.18 (m, 4H), 7.13 - 7.06 (m, 2H), 4.59 (s, 2H), 2.11 (s, 3H), 2.03 (s, 3H). ¹³C NMR (101 MHz, CDCl₃) δ 142.98, 141.50, 136.23, 136.02, 135.45, 130.32, 129.95, 129.46, 129.25, 127.48, 125.94, 125.76, 33.25, 19.98, 15.62.

### (2,2'-Dichloro-[1,1'-biphenyl]-3-yl)methanol (23)

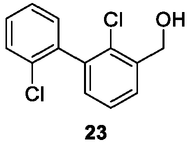

(3-Bromo-2-chlorophenyl)methanol (2.00 g, 9.03 mmol, 1.00 eq.), 2-(2-chlorophenyl)-4,4,5,5-tetramethyl-1,3,2-dioxaborolane (2.37 g, 9.93 mmol, 1.10 eq.), and K₂CO₃ (4.12 g, 29.8 mmol, 3.30 eq.) were placed in a 100 mL Schlenk flask and dissolved in water (17 mL) and 1,4-dioxane (25 mL). The two phases were degassed with argon for 1 h, after which Pd(dppf)Cl₂*CH₂Cl₂ (369 mg, 452 µmol, 5 mol%) was added under argon countercurrent. The sealed flask was heated to 100 °C and stirred at this temperature for 14.5 h before cooling to room temperature and filtering through Celite^{®} 545 with EtOAc. The phases were separated in a separatory funnel. The organic phase was washed with saturated NaHCO₃ solution and saturated NaCl solution. The combined aqueous phases were extracted three times with EtOAc, then the combined organic phases were dried over Na₂SO₄, filtered, and the solvent was evaporated under reduced pressure. The crude product was adsorbed onto silica gel and purified by column chromatography on SiO₂ using a Biotage Select system (eluent: EtOAc in petroleum ether, 0% to 50% gradient). After removal of the solvent *in vacuo,* (2,2'-dichloro-[1,1'-biphenyl]-3-yl)methanol (**23**, 2.01 g, 7.94 mmol, **88%**) was obtained as a slightly yellow oil.

R_{f}=0,31 (Cyclohexan:EtOAc 4:1); ¹H NMR (400 MHz, CDCl₃) δ 7.55 (d, J = 7.6 Hz, 1H), 7.51 - 7.45 (m, 1H), 7.40 - 7.27 (m, 3H), 7.28 - 7.23 (m, 1H), 7.23 - 7.18 (m, 1H), 4.85 (d, J = 3.9 Hz, 2H), 2.07 (t, J = 5.7 Hz, 1H). ¹³C NMR (101 MHz, CDCl₃) δ 139.02, 138.90, 138.51, 133.66, 132.03, 131.30, 130.44, 129.59, 129.41, 128.31, 126.73, 126.69, 63.40.

### 3-(Bromomethyl)-2,2'-dichloro-1,1'-biphenyl (24)

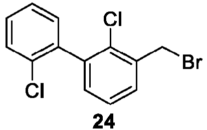

A 100 mL round-bottomed flask was alternately heated under high vacuum (10⁻³ mbar) and flushed with argon three times. Compound **23** (1.30 g, 5.14 mmol, 1.00 eq.) was dissolved in absolute CH₂Cl₂ (22 mL) and cooled to 0 °C. PBr₃ (585 µL, 1.67 g, 6.16 mmol, 1.20 eq.) was slowly added dropwise, and the solution was stirred for 4 h at 0 °C. Silica gel was added, and the solvent was removed under vacuum. The crude product was purified by column chromatography on SiO₂ using a Biotage Select system (eluent: EtOAc in petroleum ether, 0% to 20% gradient). After removal of the solvent *in vacuo,* 3-(bromomethyl)-2,2'-dichloro-1,1'-biphenyl (**24**, 1.38 g, 4.35 mmol, 85%) was obtained as a colorless solid.

R_{f}=0.62 (Cyclohexane:EtOAc 4:1); ¹H NMR (400 MHz, CDCl₃) δ 7.53 - 7.43 (m, 2H), 7.39 - 7.19 (m, 5H), 4.71 (d, J = 10.2 Hz, 1H), 4.63 (d, J = 10.2 Hz, 1H). ¹³C NMR (101 MHz, CDCl₃) δ 139.75, 138.36, 136.08, 133.81, 133.56, 131.57, 131.22, 130.96, 129.61, 129.53, 126.85, 126.73, 31.26.

### 2-Bromo-1-iodo-3-methylbenzene (25)

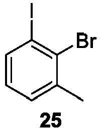

2-Bromo-3-methylaniline (20.0 g, 108 mmol, 1.00 eq.) in 1 M HCl (380 ml) was placed in a 1 L three-necked flask fitted with a dropping funnel and cooled to 0 °C. NaNO₂ (8.90 g, 129 mmol, 1.20 eq.) dissolved in H₂O (122 ml) was added dropwise over 30 min, during which time the aniline dissolved completely. The mixture was stirred for a further hour at 0 °C, after which KI (35.7 g, 215 mmol, 2.00 eq.) dissolved in H₂O (122 ml) was slowly added dropwise over 35 min. The solution turned reddish-brown, and a black solid precipitated. The reaction mixture was then warmed to room temperature and stirred for a further 16 h, after which the solid had redissolved. CH₂Cl₂ and Na₂S₂O₃ were added, decolorizing the aqueous phase. The phases were transferred to a separatory funnel with CH₂Cl₂, separated, and the aqueous phase extracted three times with CH₂Cl₂. The combined organic phases were dried over Na₂SO₄, filtered, and the solvent removed *in vacuo.* The crude product was adsorbed onto silica gel and purified by column chromatography on SiO₂ using a Biotage Select system (eluent: cyclohexane). The still slightly impure product was finally purified by vacuum distillation (boiling point 78 °C at 1.8*10⁻² bar). 2-bromo-1-iodo-3-methylbenzene (**25**, 18.5 g, 62.3 mmol, **58%**) was obtained as a colorless liquid.

¹H NMR (400 MHz, CDCl₃) δ 7.71 (d, J = 7.9 Hz, 1H), 7.19 (d, J = 7.5 Hz, 1H), 6.91 (t, J = 7.7 Hz, 1H), 2.51 (s, 3H). ¹³C NMR (101 MHz, CDCl₃) δ 140.28, 138.17, 131.82, 130.35, 128.57, 102.55, 26.19.

### 2,2'-Dibromo-3-methyl-1,1'-biphenyl (26)

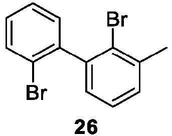

Compound **25** (2.00 g, 6.74 mmol, 1.00 eq.), (2-bromophenyl)boronic acid (1.49 g, 7.41 mmol, 1.10 eq.) and Na₂CO₃ (2.36 g, 22.2 mmol, 3.30 eq.) were placed in a 100 mL Schlenk flask and dissolved in water (12 mL) and 1,4-dioxane (19 mL). The two phases were degassed with argon for 1 h, after which Pd(PPh₃)₄ (389 mg, 337 µmol, 5 mol%) was added in countercurrent argon. The sealed flask was heated to 100 °C and stirred at this temperature for 16 h before cooling to room temperature and the phases were transferred to a separatory funnel with EtOAc and separated. The organic phase was washed with sat. NaHCO₃ solution and saturated NaCl solution. The combined aqueous phases were extracted three times with EtOAc, and the combined organic phases were then dried over Na₂SO₄, filtered, and the solvent was evaporated under reduced pressure. The crude product was adsorbed onto silica gel and purified by column chromatography on SiO₂ using a Biotage Select system (eluent: EtOAc in petroleum ether, 0% to 30% gradient). After removal of the solvent and the remaining starting material **25** under high vacuum (10⁻³ mbar), 2,2'-dibromo-3-methyl-1,1'-biphenyl (**26**, 1.60 g, 4.90 mmol, **73%**) was obtained as a colorless oil.

¹H NMR (400 MHz, CDCl₃) δ 7.65 (d, J = 7.8 Hz, 1H), 7.35 (ddd, J = 7.5, 7.5, 1.3 Hz, 1H), 7.29 - 7.14 (m, 4H), 7.08 - 7.00 (m, 1H), 2.48 (s, 3H). ¹³C NMR (101 MHz, CDCl₃) δ 143.05, 142.74, 138.78, 132.65, 131.08, 130.33, 129.31, 128.42, 127.22, 126.82, 126.08, 123.69, 24.04.

### 2,2'-Dibromo-3-(bromomethyl)-1,1'-biphenyl (27)

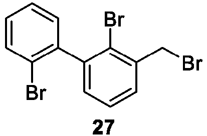

In a 25 mL round-bottomed flask, Compound **26** was dissolved in MeCN (8 mL), and NBS (1.28 g, 7.18 mmol, 1.80 eq.) was added. The solution was heated to 55 °C and irradiated with an aquarium lamp (365 nm emission) for 3.5 h. The solvent was then removed under vacuum, and the residue was adsorbed onto silica gel. Purification was achieved by column chromatography on SiO₂ using a Biotage Select system (eluent: cyclohexane). The product 2,2'-dibromo-3-(bromomethyl)-1,1'-biphenyl (**27**, 1.12 g, 2.78 mmol, **70%**) was obtained as a colorless oil.

R_{f}=0.23 (Cyclohexane); ¹H NMR (400 MHz, CDCl₃) δ 7.66 (dd, J = 8.0, 1.4 Hz, 1H), 7.50 (dd, J = 7.7, 1.7 Hz, 1H), 7.42 - 7.31 (m, 2H), 7.30 - 7.22 (m, 2H), 7.17 (dd, J = 7.6, 1.7 Hz, 1H), 4.73 (d, J = 10.3 Hz, 1H), 4.66 (d, J = 10.3 Hz, 1H).

### 2-(Benzo[c][1,2,5]oxadiazol-5-ylmethoxy)-5-chloro-4-((2-methoxyethoxy)methoxy)benzaldehyde (28)

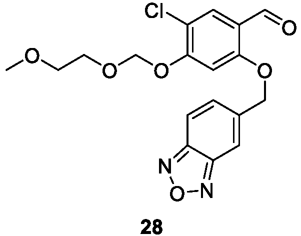

A 100 mL Schlenk flask was alternately heated under high vacuum (10⁻³ mbar) and flushed with argon three times. Compound **3** (4.60 g, 17.7 mmol, 1.00 eq.) and 5-(bromomethyl)benzo[c][1,2,5]oxadiazole (4.51 g, 21.2 mmol, 1.20 eq.) were placed therein and dissolved in DMF. Cs₂CO₃ (11.5 g, 35.29 mmol, 2.00 eq.) was added, and the suspension was stirred for 1.5 h at room temperature. Water (45 mL) was added, and the precipitate was dissolved with EtOAc. The phases were transferred to a separatory funnel and separated. The aqueous phase was extracted three times with EtOAc, and the combined organic phases were dried over Na₂SO₄, filtered, and the solvent was evaporated under reduced pressure. The crude product was adsorbed onto silica gel and purified by column chromatography on SiO₂ using a Biotage Select system (eluent: EtOAc in cyclohexane, 0% to 100% gradient). 2-(benzo[c][1,2,5]oxa-diazol-5-ylmethoxy)-5-chloro-4-((2-methoxyethoxy)methoxy)benzaldehyde (**28**, 6.93 g, 17.7 mmol, **quant**.) was obtained as a pale yellow solid.

R_{f}=0.07 (cyclohexane:EtOAc 2:1); ¹H NMR (400 MHz, CDCl₃) δ 10.36 (s, 1H), 7.93 (s, 1H), 7.91 (d, J = 9.3 Hz, 1H), 7.87 (s, 1H), 7.48 (d, J = 9.2 Hz, 1H), 6.98 (s, 1H), 5.40 (s, 2H), 5.24 (s, 2H), 3.89 - 3.82 (m, 2H), 3.57 - 3.49 (m, 2H), 3.35 (s, 3H). ¹³C NMR (101 MHz, CDCl₃) δ 186.79, 160.14, 158.86, 149.10, 148.93, 139.68, 130.85, 130.41, 120.22, 117.54, 117.22, 114.23, 100.91, 94.22, 71.40, 69.95, 68.45, 59.10.

### 2-(Benzo[c][1,2,5]oxadiazol)5-ylmethoxy)-5-chloro-4-hydroxybenzaldehyde (29)

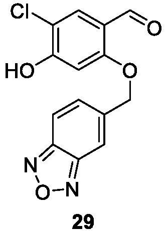

In a 1 L round-bottomed flask, Compound **28** (5.50 g, 14.0 mmol, 1.00 eq.) was placed in air and dissolved in CH₂Cl₂ (275 mL) and TFA (275 mL). The mixture was stirred at room temperature for 5.5 h, after which EtOAc (250 mL) was added. The resulting precipitate was filtered, washed with copious amounts of EtOAc, and dried under high vacuum (10⁻³ mbar). 2-(benzo[c][1,2,5]oxadiazol-5-ylmethoxy)-5-chloro-4-hydroxybenzaldehyde (**29**, 4.00 g, 13.1 mmol, **94%**) was obtained as a yellow solid.

¹H NMR (400 MHz, DMSO) δ 11.53 (s, 1H), 10.22 (s, 1H), 8.19 (dd, J = 1.3, 1.3 Hz, 1H), 8.12 (dd, J = 9.3, 1.0 Hz, 1H), 7.71 (dd, J = 9.3, 1.3 Hz, 1H), 7.68 (s, 1H), 6.80 (s, 1H), 5.38 (s, 2H). ¹³C NMR (101 MHz, DMSO) δ 186.57, 160.19, 159.84, 148.85, 148.60, 141.37, 132.38, 129.66, 117.92, 116.45, 113.47, 113.00, 101.55, 69.03.

### Preparation of the Compounds according to the invention

### Example 1 - Compound 20 (FS-594)

### Step 1 - Preparation of (16R,19R,22R)-22-((R)-4-(4,7-bis(2-(tert-butoxy)-2-oxoethyl)-1,4,7-triazonan-1-yl)-5-(tert-butoxy)-5-oxopentanamido)-1-(4-((2-bromo-[1,1'-biphenyl]-3-yl)methoxy)-5-chloro-2-((5-(methylsulfonyl)pyridin-3-yl)methoxy)phenyl)-12,15,18,21-tetraoxo-16,19-bis(sulfomethyl)-5,8-dioxa-2,11,14,17,20-pentaazatricosane-23-sulfonic acid (19)

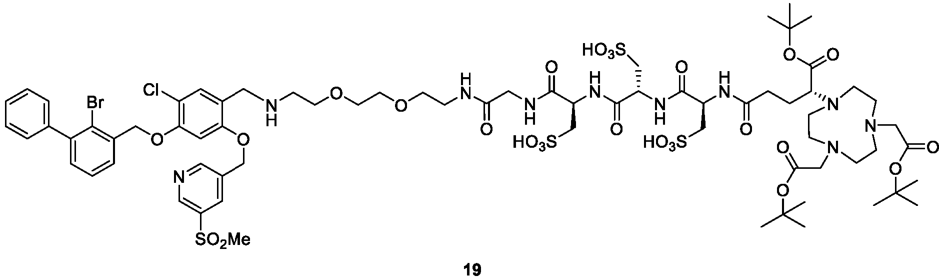

Compound **18** (8.20 mg, 6.67 µmol, 1.00 eq.) and (*R*)-NODA-GA(*^{t}*Bu)₃ (7.25 mg, 13.3 µmol, 2.00 eq.) were placed in a 25 mL round-bottomed flask and dissolved in DMF (500 µL). DIPEA (4.65 µL, 26.7 µmol, 4.00 eq.) was added, and the solution was cooled to 0 °C. Oxyma Pure (1.90 mg, 13.3 µmol, 2.00 eq.) and EDC (2.07 mg, 13.3 µmol, 2.00 eq.) were added. The solution was stirred for 16 h, slowly warming to room temperature. The solvent was then concentrated under reduced pressure, and the residue was dissolved in a 1:1 mixture of water and MeCN. The crude product was purified by HPLC (System B). (16*R*,19*R*,22*R*)-22-((*R*)-4-(4,7-bis(2-(*tert-*butoxy)-2-oxoethyl)-1,4,7-triazonan-1-yl)-5-(*tert-*butoxy)-5-oxopentanamido)-1-(4-((2-bromo-[1,1'-biphenyl]-3-yl)methoxy)-5-chloro-2-((5-(methylsulfonyl)pyridin-3-yl)methoxy)phenyl)-12,15,18,21-tetraoxo-16,19-bis(sulfomethyl)-5,8-dioxa-2,11,14,17,20-pentaazatricosane-23-sulfonic acid (**19**) was obtained as a white solid.

Mass calculated for [M - H]-: m/z = 1751.46; measured: m/z = 1751.27.

### Step 2 - Preparation of 2,2'-(7-((16R,19R,22R,27R)-1-(4-((2-bromo-[1,1'-biphenyl]-3-yl)methoxy)-5-chloro-2-((5-(methylsulfonyl)pyridin-3-yl)methoxy)phenyl)-27-carboxy-12,15,18,21,24-pentaoxo-16,19,22-tris(sulfomethyl)-5,8-dioxa-2,11,14,17,20,23-hexaazaheptacosan-27-yl)-1,4,7-triazonane-1,4-diyl)diacetic acid (20)

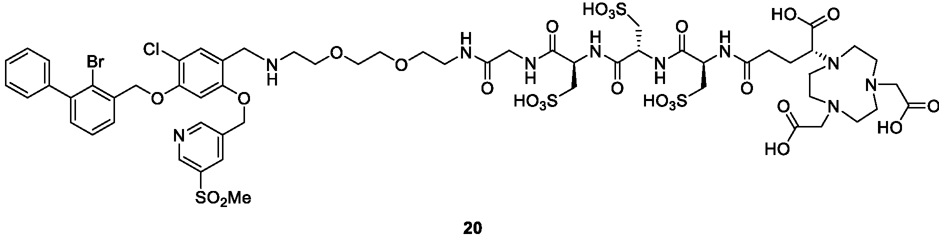

Compound **19** (from the previous reaction, 1.00 eq.) and Yb(OTf)₃ (124 mg, 200 µmol, 30.0 eq.) were placed in a 10 mL round-bottomed flask and suspended in CH₃NO₂ (1.0 mL). The mixture was heated to 45 °C and stirred at constant temperature for 48 h. The solvent was then removed under vacuum, and the residue was dissolved in water. The crude product was purified by HPLC (System A). 2,2'-(7-((16*R*,19*R*,22*R*,27*R*)-1-(4-((2-bromo-[1,1'-biphenyl]-3-yl)methoxy)-5-chloro-2-((5-(methylsulfonyl)pyridin-3-yl)methoxy)phenyl)-27-carboxy-12,15,18,21,24-pentaoxo-16,19,22-tris(sulfomethyl)-5,8-dioxa-2,11,14,17,20,23-hexaazaheptacosan-27-yl)-1,4,7-triazonane-1,4-diyl)diacetic acid (**20**, 3.07 mg, 1.93 µmol, **29% over 2 steps**) was obtained as a white solid.

Mass calculated for [M - H]-: m/z = 1583.28; measured: m/z = 1583.27.

### Example 2 - Compound 30

### 2,2'-(7-((16R,19R,22R,27R)-27-Carboxy-1-(5-chloro-4-((2,2'-dimethyl-[1,1'-biphenyl]-3-yl)methoxy)-2-((5-(methylsu.ffonyl)pyridin-3-yl)methoxy)phenyl)-12,15,18,21,24-pentaoxo-16,19,22-tris(su.ffomethyl)-5,8-dioxa-2,11,14,17,20,23-hexaazaheptacosan-27-yl)-1,4,7-triazonane-1,4-diyl)diacetic acid

According to the procedures for the preparation of Compound 20 but starting from compound 22 the compound 30 can be obtained.

### Example 3 - Compound 31

### 2,2'-(7-((16R,19R,22R,27R)-27-Carboxy-1-(5-chloro-4-((2,2'-dichloro-[1,1"-biphenyl]-3-yl)methoxy)-2-((5-(methylsulfonyl)pyridin-3-yl)methoxy)phenyl)-12,15,18,21,24-pentaoxo-16,19,22-tris(su.ffomethyl)-5,8-dioxa-2,11,14,17,20,23-hexaazaheptacosan-27-yl)-1,4,7-triazonane-1,4-diyl)diacetic acid

According to the procedures for the preparation of Compound 20 but starting from compound 24 the compound 31 can be obtained.

### Example 4 - Compound 32

### 2,2'-(7-((16R,19R,22R,27R)-27-carboxy-1-(5-chloro-4-((2,2'-dibromo-[1,1'-biphenyl]-3-yl)methoxy)-2-((5-(methylsu.ffonyl)pyridin-3-yl)methoxy)phenyl)-12,15,18,21,24-pentaoxo-16,19,22-tris(sulfomethyl)-5,8-dioxa-2,11,14,17,20,23-hexa-azaheptacosan-27-yl)-1,4,7-triazonane-1,4-diyl)diacetic acid

According to the procedures for the preparation of Compound 20 but starting from compound 27 the compound 32 can be obtained.

### Example 5 - Compound 33

### 2,2'-(7-((16R,19R,22R,27R)-1-(2-(benzo[c][1,2,5]oxadiazol-5-ylmethoxy)-5-chloro-4-((2,2'-dimethyl-[1,1'-biphenyl]-3-yl)methoxy)phenyl)-27-carboxy-12,15,18,21,24-pentaoxo-16,19,22-tris(sulfomethyl)-5,8-dioxa-2,11,14,17,20,23-hexaazaheptacosan-27-yl)-1,4,7-triazonane-1,4-diyl)diacetic acid

According to the procedures for the preparation of Compound 20, but starting from compound 22 and reaction with compound 29, the compound 33 can be obtained.

### Example 6 - Compound 34

### 2,2'-(7-((16R,19R,22R,27R)-1-(2-(benzo[c][1,2,5]oxadiazol-5-ylmethoxy)-5-chloro-4-((2,2'-dichloro-[1,1'-biphenyl]-3-yl)methoxy)phenyl)-27-carboxy-12,15,18,21,24-pentaoxo-16,19,22-tris(sulfomethyl)-5,8-dioxa-2,11,14,17,20,23-hexaazaheptacosan-27-yl)-1,4,7-triazonane-1,4-diyl)diacetic acid

According to the procedures for the preparation of Compound 20, but starting from compound 24 and reaction with compound 29, the compound 34 can be obtained.

### Example 7 - Compound 35

### 2,2'-(7-((16R,19R,22R,27R)-1-(2-(benzo[c][1,2,5]oxadiazol-5-ylmethoxy)-5-chloro-4-((2,2'-dibromo-[1,1'-biphenyl]-3-yl)methoxy)phenyl)-27-carboxy-12,15,18,21,24-pentaoxo-16,19,22-tris(sulfomethyl)-5,8-dioxa-2,11,14,17,20,23-hexaazaheptacosan-27-yl)-1,4,7-triazonane-1,4-diyl)diacetic acid

According to the procedures for the preparation of Compound 20, but starting from compound 27 and reaction with compound 29, the compound 35 can be obtained.

### Example 8 - Compound 36

### 2,2'-(7-((16R,19R,22R,27R)-1-(2-(benzo[c][1,2,5]oxadiazol-5-ylmethoxy)-4-((2-bromo-[1,1'-biphenyl]-3-yl)methoxy)-5-chlorophenyl)-27-carboxy-12,15,18,21,24-pentaoxo-16,19,22-tris(sulfomethyl)-5,8-dioxa-2,11,14,17,20,23-hexaazaheptacosan-27-yl)-1,4, 7-triazonane-1,4-diyl)diacetic acid

According to the procedures for the preparation of Compound 20, but starting from compound 9 and reaction with compound 29, the compound 36 can be obtained.

### Example 9

### Labeling of Compound 20 with Cu-64

The mixture of the ligand **20** (4 nmol from a 1 mM solution in Water) with approximately 100 MBq ⁶⁴Cu-solution was prepared in a HEPES solution in a 1.5 ml Protein LoBind^{®} Eppendorf tube. Incubation took place at 300 rpm and 50 °C for 10 min. The tube was then cooled for three minutes, centrifuged, and a small volume was applied to iTLC-SG chromatography paper (stationary phase). A 0.1 M citrate solution (adjusted to pH 4 with 1 M NaOH) was used as the mobile phase. The chromatography paper was analyzed using a radiochromatography paper scanner (Rita Star, Elysia-Raytest GmbH, Straubenhardt). The labeling efficiency was determined by the ratio of the integrated areas of the unbound radiometal (R_{f} = 0) and the radiometal complex (R_{f} = 0.8).

The Cu-64 labeled compound 20 (FS-594) is also referred to as [⁶⁴Cu]Cu-FS-594.

### Example 10 - In Vitro Experiments - Radioligand binding assay

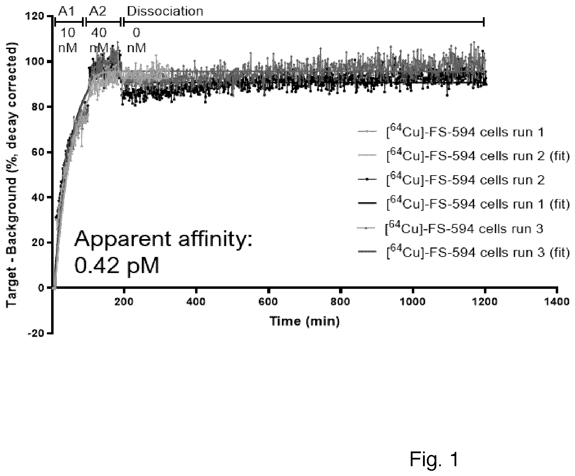

Radioligand binding assays were performed on a live human PC3 cells overexpressing human PD-L1 in sixfold and three experiments were performed - two association (A1/A2, 10 and 40 nM of tracer) phases are followed by a dissociation phase (medium only) (see Figure 1). Data show a complete internalisation of the [⁶⁴Cu]Cu-FS-594 complex without any efflux over 16 hours.

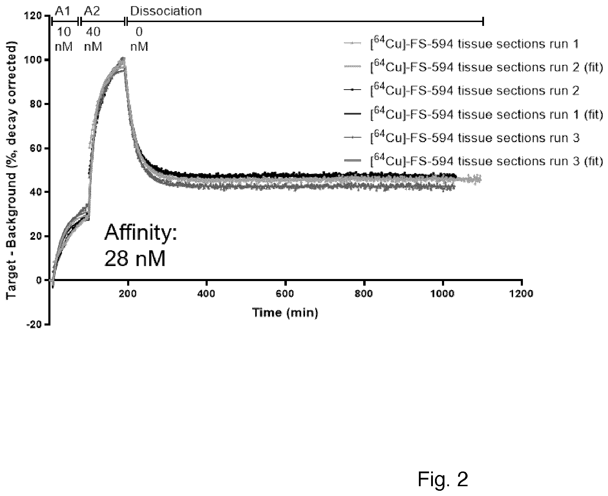

Further radioligand binding assays were performed on xenograft tissue sections from PC3 cells overexpressing human PD-L1 and three experiments were performed - two association (A1/A2, 10 and 40 nM of tracer) phases are followed by a dissociation phase (medium only) (see Figure 2). Data show a fast dissociation within the first hour and lack of internalisation of the [⁶⁴Cu]Cu-FS-594 complex.

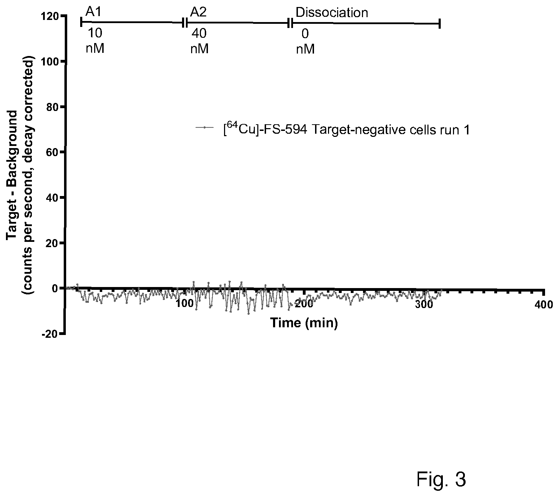

Another radioligand binding assay was performed on live PC3 cells lacking human PD-L1 expression (see Figure 3). The data shows an absence of any binding (signal increase over time, proving the specificity of the signal observed in figures 1 and 2.

[⁶⁴Cu]Cu-FS-594 shows good water solubility with a Log *D*_{7.4} value of -2.93± 0.03. On living and PD-L1-overexpressing cells, this radioligand shows a high binding affinity with an apparent *K*_{D} of 27.4 pM.

### Example 11 - In Vivo Experiments

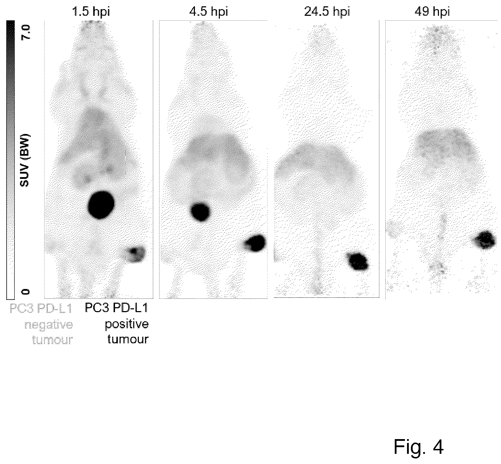

Figure 4 shows PET images of [⁶⁴Cu]Cu-FS-594 in mice bearing xenografts from PC3 PD-L1 overexpressing cells (right leg) and PC3 target-negative cells (left leg). Maximum intensity projections are given at indicated timepoints, following injection of 10.7 MBq [⁶⁴Cu]Cu-FS-594. High and selective uptake in PD-L1 positive tumor tissue can be observed, starting at 1.5 h and plateauing from 4.5 h to 49 h.

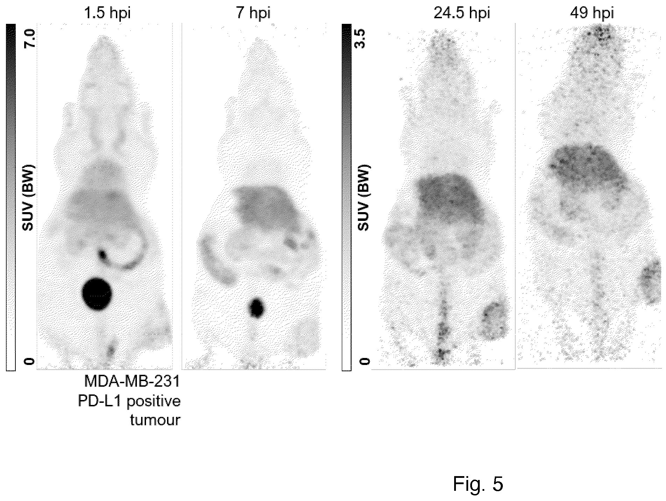

Figure 5 shows PET images of [⁶⁴Cu]Cu-FS-594 in mice bearing xenografts from MDA-MB-231 cell that exhibit a natural PD-L1 expression. Maximum intensity projections are provided at indicated timepoints, following injection of 8.7 MBq [⁶⁴Cu]Cu-FS-594. Selective uptake in PD-L1 positive tumor tissue can be observed starting from 1.5 h onwards, reaching a plateau from 24.5 h onwards. Table 1 shows various data from the PET imaging, e.g tumor uptake, contrast between tumor entities and tumor-to-muscle ratio.

**Table 1**

| | 1.5 hpi | | | 4.5 hpi | | |
|---|---|---|---|---|---|---|
| Tissue | SUVₘₐₓ | contrast target-positive/negative tumor (%) | Ratio target-positive tumor vs. muscle | SUVₘₐₓ | contrast target-positive/negative tumor (%) | Ratio target-positive tumor vs. muscle |
| PC3 PD-L1 tumor | 8.92 | 546.38 | | 13.8 | 1121.4 | |
| PC3 Mock tumor | 1.38 | | | 1.13 | | |
| | | | | | | |
| muscle | 0.62 | | 14.39 | 0.31 | | 44.52 |
| | | | | | | |

| | 24.5 hpi | | | 49 hpi | | |
|---|---|---|---|---|---|---|
| Tissue | SUVₘₐₓ | contrast target-positive/negative tumor (%) | Ratio target-positive tumor vs. muscle | SUVₘₐₓ | contrast target-positive/negative tumor (%) | Ratio target-positive tumor vs. muscle |
| PC3 PD-L1 tumor | 10.53 | 403.83 | | 9.44 | 439.43 | |
| PC3 Mock tumor | 2.09 | | | 1.75 | | |
| | | | | | | |
| muscle | 0.31 | | 33.97 | 0.72 | | 34.96 |

In summary, it can be stated that dead PC3 PD-L1 overexpressing cells or frozen sections of PC3 PD-L1 positive mouse tumors show similar rapid kinetics, but no internalization. In tumor-bearing mice, the compound showed rapid, selective and high accumulation in PC3 PD-L1-positive tumor tissue, resulting in a very good contrast to PD-L1-negative tissue. Furthermore, the radioligand shows rapid and predominantly renal excretion and only low uptake in the liver. The accumulation is still very high in the tumor tissue after 73 h, which confirms the in vitro observed internalization of the radioligand into cells. Selective tumor uptake can be reliably blocked by co-injection with 100 nmol of unlabeled inventive compounds. Such excellent contrast has not yet been described for other non-peptidic, low-molecular weight PD-L1 radioligands and therefore demonstrates the high potential of the compounds according to the invention.

### Literature

1. Aktoudianakis, E.A., Todd; Cho, Aesop; Du, Zhimin; Graupe, Michael, PD-1/PD-L1 Inhibitors. WO 2018/195321 A1, 2018.
2. Stephen E. Webber, R.J.A., Immune Checkpoint Inhibitors Compositions and Methods Thereof. WO2018/045142 A1, 2018.
3. Bi, Y.D., Bruce D., Fan, Yi; Moore, Christopher Brooks; Nguyen, Duyan, Substituted 1,1 '-Biphenyl Compounds, Analogues Thereof, and Methods Using Same, I. Arbutus Biopharma, Editor. 2019.
4. Parcella; Mark George Saulnier; Li-Qiang Sun; Alan Xiangdong Wang; Ningning Xu; Juliang Zhu; Paul Michael Scola, K.-S.Y.T.P.C.D.B.F.K.A.G.-Y.P.H.D.R.L.Z.M.E.M.K.E., Compounds useful as immunomodulators. US 2017/0107202 A1, 2017.
5. Feng, Z.C., Xiaoguang; Yang, Yang; Zhou, Chuan; Ji, Ming; Jin, Xiaofeng; Xue, Nina; Zheng, Yi; Chen, Hao; Li, Ling, Phenylate Derivative, Preparation Method therefore, and pharmaceutical composition, and uses thereof. WO 2017/202276 A1, 2017.
6. Odegard, J.M., et al., Oral PD-L1 inhibitor GS-4224 selectively engages PD-L1 high cells and elicits pharmacodynamic responses in patients with advanced solid tumors. Journal for ImmunoTherapy of Cancer, 2024. 12(4): p. e008547.
7. Koblish, H.K., et al., Characterization of INCB086550: A Potent and Novel Small-Molecule PD-L1 Inhibitor. Cancer Discovery, 2022. 12(6): p. 1482-1499.
8. Park, J.-J., et al., Checkpoint inhibition through small molecule-induced internalization of programmed death-ligand 1. Nature Communications, 2021. 12(1): p. 1222.
9. Wang, T., et al., Discovery of Small-Molecule Inhibitors of the PD-1/PD-L1 Axis That Promote PD-L1 Internalization and Degradation. J Med Chem, 2022. 65(5): p. 3879-3893.
10. Guzik, K., et al., Development of the Inhibitors that Target the PD-1/PD-L1 Interaction-A Brief Look at Progress on Small Molecules, Peptides and Macrocycles. Molecules, 2019. 24(11).
11. Bridoux, J., et al., Anti-Human PD-L1 Nanobody for Immuno-PET Imaging: Validation of a Conjugation Strategy for Clinical Translation. Biomolecules, 2020. 10(10): p. 1388.
12. Fu, R., et al., Antibody Fragment and Affibody ImmunoPET Imaging Agents: Radiolabelling Strategies and Applications. ChemMedChem, 2018. 13(23): p. 2466-2478.
13. Niemeijer, A.N., et al., Whole body PD-1 and PD-L1 positron emission tomography in patients with non-small-cell lung cancer. Nature Communications, 2018. 9(1): p. 4664.
14. Bensch, F., et al., 89Zr-atezolizumab imaging as a non-invasive approach to assess clinical response to PD-L1 blockade in cancer. Nature Medicine, 2018.
15. Krutzek, F., K. Kopka, and S. Stadlbauer, Development of Radiotracers for Imaging of the PD-1/PD-L1 Axis. Pharmaceuticals, 2022. 15(6): p. 747.
16. Kuan, H., et al., Developing native peptide-based radiotracers for PD-L1 PET imaging and improving imaging contrast by pegylation. Chemical Communications, 2019. 55(29): p. 4162-4165.
17. Lesniak, W.G., et al., Development of [(18)F]FPy-WL12 as a PD-L1 Specific PET Imaging Peptide. Molecular Imaging, 2019. 18: p. 1536012119852189-1536012119852189.
18. De Silva, R.A., et al., Peptide-Based (68)Ga-PETRadiotracer for Imaging PD-L1 Expression in Cancer. Mol Pharm, 2018. 15(9): p. 3946-3952.
19. Chatterjee, S., et al., Rapid PD-L1 detection in tumors with PET using a highly specific peptide. Biochemical and Biophysical Research Communications, 2017. 483(1): p. 258-263.
20. Kumar, D., et al., Peptide-based PET quantifies target engagement of PD-L1 therapeutics. J Clin Invest, 2018.
21. Zhou, X., et al., First-in-human evaluation of a PD-LI-binding peptide radiotracer in non-small cell lung cancer patients with PET. Journal of Nuclear Medicine, 2021: p. jnumed.121.262045.
22. Miao, Y., et al., One-Step Radiosynthesis and Initial Evaluation of a Small Molecule PET Tracer for PD-L1 Imaging. Bioorganic & Medicinal Chemistry Letters, 2020: p. 127572.
23. Lv, G., et al., Promising potential of a 18F-labelled small-molecular radiotracer to evaluate PD-L1 expression in tumors by PET imaging. Bioorganic Chemistry, 2021. 115: p. 105294.
24. Huang, Y., et al., Development and Preclinical Evaluation of [68Ga]BMSH as a New Potent Positron Emission Tomography Tracer for Imaging Programmed Death-Ligand 1 Expression. Pharmaceuticals, 2023. 16(10): p. 1487.
25. Xu, L., et al., Design, Synthesis, and Biological Evaluation of a Small-Molecule PET Agent for Imaging PD-L1 Expression. Pharmaceuticals, 2023. 16(2): p. 213.
26. Zhu, D., et al., Synthesis and preliminary biological evaluation of a novel 99mTc-labeled small molecule for PD-L1 imaging. Bioorganic & Medicinal Chemistry Letters, 2023: p. 129496.
27. Lu, C., et al., Design, synthesis, and biological evaluation of a 99mTc-labeled small-molecule tracer for PD-L1 imaging. New Journal of Chemistry, 2024. 48(16): p. 7300-7307.
28. Yang, H., et al., Development of small-molecular-based radiotracers for PET imaging of PD-L1 expression and guiding the PD-L1 therapeutics. European Journal of Nuclear Medicine and Molecular Imaging, 2024.
29. Feng, Z.Y., Yang, Iodine isotope-labeled benzyl phenyl ether derivative, and preparation method and medicine thereof. Composition and use. CN 111662270 A, 2020.
30. Huck, B.R., L. Kötzner, and K. Urbahns, Small Molecules Drive Big Improvements in Immuno-Oncology Therapies. Angewandte Chemie International Edition, 2018. 57(16): p. 4412-4428.
31. Lv, G., et al., Synthesis and immunotherapy efficacy of a PD-L1 small-molecule inhibitor combined with its 131I-iodide labelled isostructural compound. Bioorganic Chemistry, 2024. 153: p. 107810.

### List of abbreviations

- dppf: 1,1'-Bis(diphenylphosphino)ferrocene
- dba: dibenzylideneacetone
- TFA: trifluoro acetic acid
- HEPES: N-2-hydroxyethylpiperazine-N-2-ethanesulphonic acid
- DMSO: dimethyl sulphoxide
- EtOAc: ethyl acetate
- DMF: dimethyl formamide
- EtI: ethyliodide
- HCl: hydrochloric acid
- MeCN: acetonitrile
- HPLC: high performance liquid chromatography
- EDC: ethylene dichloride
- PET: polyethylene terephthalate
- THF: tetrahydrofurane
- DIPEA: diisopropylethylamine
- MEM: methoxymethyl
- MeOH: methanol
- Me: methyl
- Et: ethyl
- EtOH: ethanol
- BuOH: butanol
- PPh₃: triphenylphosphane
- Boc: tert-Butyloxycarbonyl
- Fmoc: fluorenylmethoxycarbonyl
- PET: Positron Emission Tomography
- SPECT: Single Photon Emission Computed Tomography

## Claims

1. A compound of general formula I 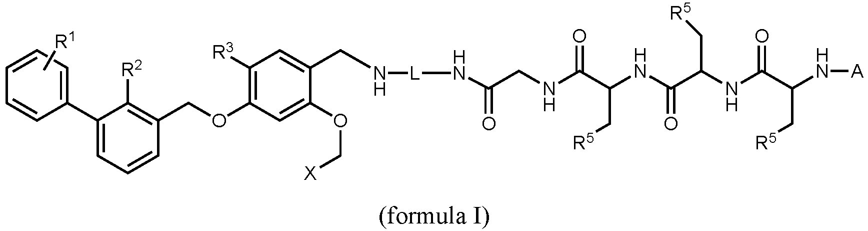 wherein
R¹ is selected from the group consisting of H, halogen, C₁-C₄ alkyl and C₁-C₄ alkoxy;
R² is selected from the group consisting of H, halogen, C₁-C₄ alkyl and C₁-C₄ alkoxy;
R³ is selected from the group consisting of H, halogen, C₁-C₄ alkyl and C₁-C₄ alkoxy;
X is a group of formula X1 or a group of formula X2
| | |
|---|---|
| 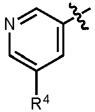 | 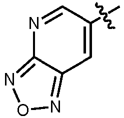 |
| X1 | X2 |
R⁴ is selected from the group consisting of -S(O)₂-C₁-C₄ alkyl, halogen and CN;
R⁵ are independently of each other selected from the group consisting of -S(O)₂OH, -P(O)(OH)₂ and -NH₂;
L is a linker selected from the group consisting of
| | |
|---|---|
| 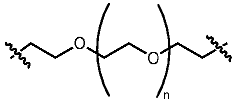 | 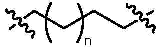 |
| L1 | L2 |
| 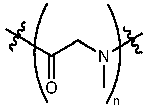 | 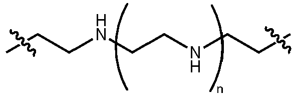 |
| L3 | L4 |
A is a chelator selected from the group consisting of
| | | |
|---|---|---|
| 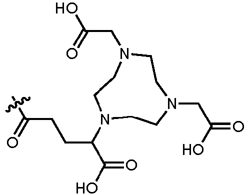 | 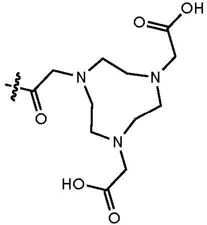 | 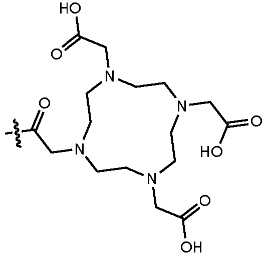 |
| A1 | A2 | A3 |
| 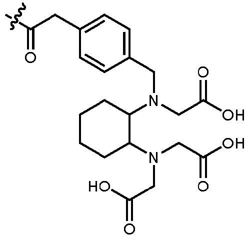 | 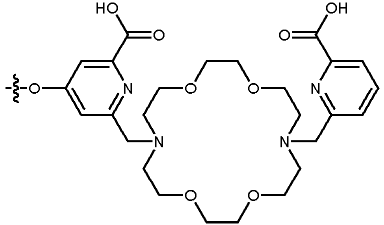 | |
| A4 | A5 | |
n is an integer from 1 to 5.

2. The compound according to claim 1, wherein X is X1.

3. The compound according to any of the preceding claims, wherein R⁵ is -S(O)₂OH.

4. The compound according to any of the preceding claims, wherein A is A1.

5. The compound according to any of the preceding claims, wherein R¹, R² and R³ are independently of each other halogens and L is L1 with n = 1.

6. The compound according to any of the preceding claims, wherein R¹ is H, R² is chlorine or bromine, R³ is chlorine or bromine, R⁴ is -S(O)₂-CH₃ and R⁵ is -S(O)₂OH.

7. The compound according to any of the preceding claims, wherein the compound is 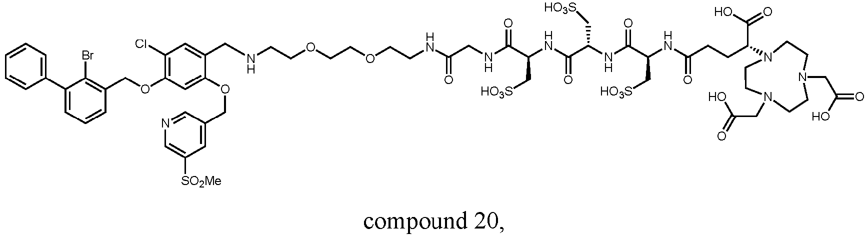 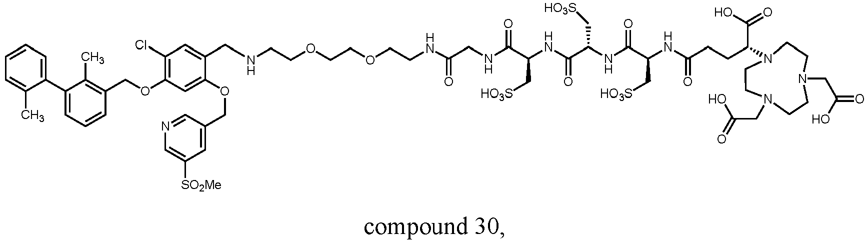 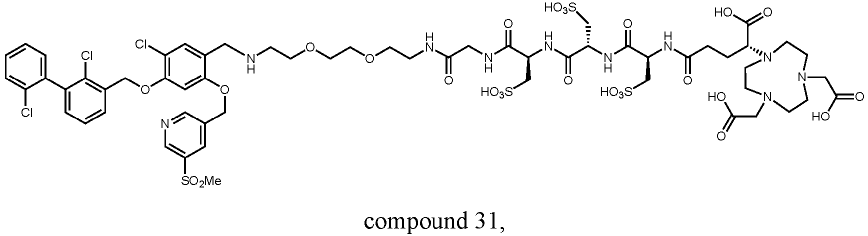 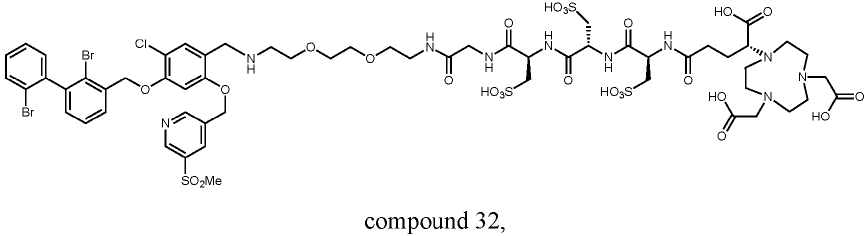 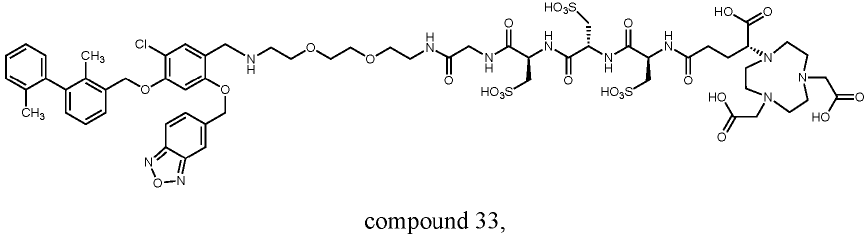 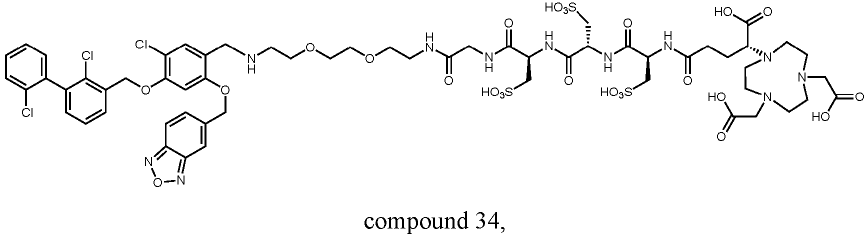 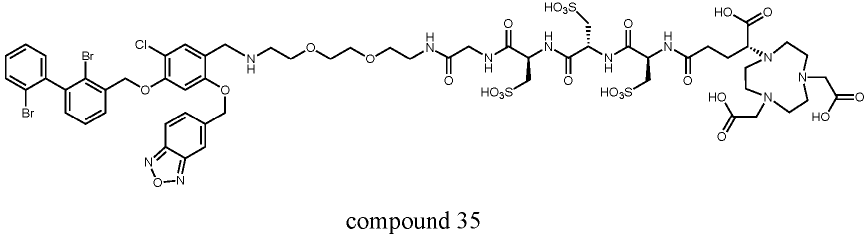 and

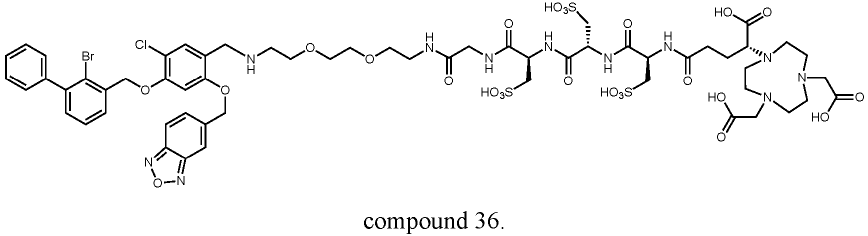

8. Complex consisting of a compound according to any of claims 1 to 7 and a radioactive metal ion or Al[¹⁸F]F²⁺.

9. Complex according to claims 7, wherein the radioactive metal ion is selected from the group consisting of [⁶¹Cu]Cu²⁺, [⁶⁴Cu]Cu²⁺, [⁶⁷Cu]Cu²⁺, [⁶⁷Ga]Ga³⁺, [⁶⁸Ga]Ga³⁺, [¹⁷⁷Lu]Lu³⁺, [¹⁴⁹Tb]Tb³⁺, [¹⁵⁵Tb]Tb³⁺, [¹⁵²Tb]Tb³⁺, [¹⁶¹Tb]Tb³⁺, [²²⁵Ac]Ac³⁺, [²²⁶Ac]Ac³⁺, [¹³²La]La³⁺, [¹³³La]La³⁺, [¹³⁵La]La³⁺, [¹¹¹In]In³⁺, [⁸⁶Y]Y³⁺, [⁹⁰Y]Y³⁺, [²¹²Bi]Bi³⁺, [²¹³Bi]Bi³⁺, [⁴³Sc]Sc³⁺, [⁴⁴Sc]Sc³⁺, [⁴⁷Sc]Sc³⁺, [²⁰³Pb]Pb³⁺ and [²¹²Pb]Pb³⁺.

10. Complex according to claim 6 or 7, wherein the radioactive metal ion [⁶⁴Cu]Cu²⁺ or [⁶⁸Ga]Ga³⁺.

11. A method for the preparation of compounds of general formula I, wherein a Compound of general formula XVIII-a 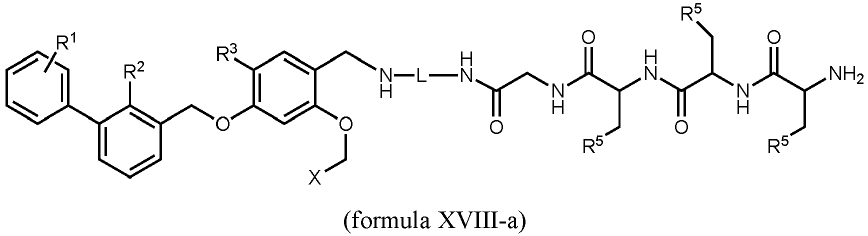
wherein R¹, R², R³, R⁴, R⁵, L, X and n have the meanings given in claim 1,
is reacted with HO-A,
wherein A has the meanings given in claim 1.

12. The compound according to any of claims 1 to 7 or complex according to claims 8 to 10 for use as medicinal drug.

13. The compound according to any of claims 1 to 7 or complex according to claims 8 to 10 for use as medicinal drug for the diagnosis and therapy of diseases, into which the Programmed Death Receptor Ligand 1 (PD-L1) is involved.

14. The compound or complex according to any of claim 12 or 13, wherein the medicinal drug is for the diagnosis and therapy of non-small cell lung carcinoma.

15. Use of a compound according to any of claims 1 to 7 in the preparation of a radiopharmaceutical agent.
